# EUROPEAN PATENT APPLICATION

(11) **EP 4 759 368 A1**
(43) Date of publication of application: **17.06.2026**
(21) Application number: 24307064.6
(22) Date of filing: 10.12.2024
(51) Int. Cl.: A61P 35/00, C07D 205/04

(54) **AZETIDINE COMPOUNDS AND THEIR USE IN THERAPY**

(71) Applicant: Inventiva, 21121 Daix (FR)
(72) Inventor: The designation of the inventor has not yet been filed
(74) Representative: Cabinet Beau de Loménie

(57) **Abstract**

The present disclosure relates to compounds of formula (I): 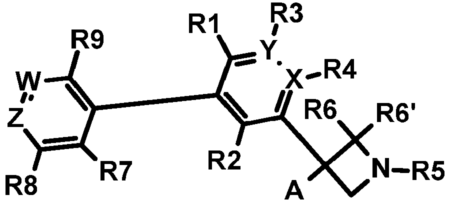 wherein A, W, X, Y, Z, R₁, R₂, R₃, R₄, R₅, R₆, R'₆, R₇, R₈ and R₉ are as defined herein. The compounds of formula (I) are useful as modulators of the Hippo signalling pathway.

## Description

### Field of the invention

The present disclosure relates to compounds that modulate the YAP/TAZ-TEAD complex, to pharmaceutical compositions containing said compounds, and to the use of said compounds as medicaments, in particular for the treatment and/or prevention of cancers and/or fibrosis related diseases.

### Background

The hippo signalling pathway has key roles in organ size control and tumor suppression. Signal transduction in the hippo signalling pathway involves a core kinase cascade, leading to YAP/TAZ phosphorylation. Physiological or pathological inactivation of the hippo signalling pathway leads to dephosphorylation and nuclear accumulation of YAP and/or TAZ. Nuclear YAP/TAZ binds to transcriptional enhanced associate domains (TEADs) to mediate target gene expression. The YAP/TAZ-TEAD complex regulates organ development and amplification of oncogenic factors in many cancers (e.g., sarcoma, lung cancer, thyroid cancer, skin cancer, ovarian cancer, colorectal cancer, prostate cancer, pancreatic cancer, head and neck cancer, oesophageal cancer, kidney cancer, urogenital cancer, liver cancer, breast cancer) and in fibrosis related diseases. There is a need to find modulators of the Hippo signalling pathway useful as medicaments.

The present invention provides new compounds that disrupt the YAP/TAZ-TEAD complex.

### Summary of the invention

In one aspect, the present disclosure relates to compounds of formula (I): 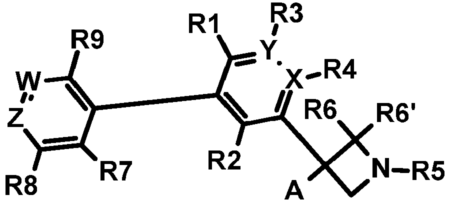 or a pharmaceutically acceptable salt, enantiomers, diastereomers, tautomers, solvates, isotope substituents, polymorphs, prodrugs or metabolites thereof, wherein:
A is a phenyl optionally substituted with 1 to 3 substituents selected from halogen, (C₁-C₆)alkyl and (C₁-C₆)alkoxy; or a 4- to 7-membered unsaturated monocyclic group comprising from 1 to 3 heteroatoms, preferably 1 or 2 heteroatoms, chosen from nitrogen, oxygen and sulfur;
W and Z are each independently CR₁₅ or N;
X and Y are each independently C or N;
R₁ is H, halogen, (C₁-C₆)alkyl or (C₁-C₆)alkoxy;
R₂ is H, halogen, (C₁-C₆)alkyl or (C₁-C₆)alkoxy;
R₃ is absent when Y is N, or R₃ is H, halogen, -OH, -COCH₃, (C₁-C₆)alkyl or (C₁-C₆)alkoxy when Y is C;
R₄ is absent when X is N, or R₄ is H, halogen, -OH, -COCH₃, (C₁-C₆)alkyl or (C₁-C₆)alkoxy when X is C;
R₅ is H, (C₁-C₆)alkyl optionally substituted with halogen, -OH, -CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, - CONHR₁₂, -CONH₂, -NR₁₂R₁₃;
R₆ and R₆' are each independently H, (C₁-C₆)alkyl or (C₃-C₆)cycloalkyl;
Or R₆ and R₆' are bond together to form a 3 or 4 membered carbocyclic ring
R₇ is H, halogen, (C₁-C₆)alkyl or (C₁-C₆)alkoxy optionally substituted with (C₁-C₆)alkoxy;
R₈ is H, halogen, (C₁-C₆)alkyl, (C₁-C₆)alkoxy, (C₁-C₆)alkoxy optionally substituted with (C₁-C₆)alkoxy,-OR₁₀, -OCH₂R₁₀, -NHR₁₀, -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen ;
R₉ is (C₁-C₆)alkyl ,(C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen,;
R₁₁ is H, -OH ,-NH₂, (C₁-C₆)alkyl, (C₁-C₆)alkoxy, and -NHR₁₄ ;
R₁₂ is H or (C₁-C₆)alkyl;
R₁₃ is H or (C₁-C₆)alkyl;
R₁₄ is (C₁-C₆)alkyl;
R₁₅ is H, halogen, (C₁-C₆)alkyl or (C₁-C₆)alkoxy.

In one aspect, the present disclosure relates to a pharmaceutical composition comprising a compound of formula (I) or a pharmaceutically acceptable salt thereof, and one or more pharmaceutically acceptable excipients.

In one aspect, the present disclosure provides a method of modulating one or more of proteins encompassed by, or related to, the Hippo pathway in a subject, comprising administering to a subject in need thereof a compound of formula (I), or a pharmaceutically acceptable salt thereof.

In one aspect, the present disclosure relates to a method of treating or preventing a cancer which comprises administering an effective amount of a compound of formula (I) or a pharmaceutically acceptable salt thereof to a subject in need thereof.

In one aspect, the present disclosure relates to a method of treating or preventing a fibrosis-related disease which comprises administering an effective amount of a compound of formula (I) or a pharmaceutically acceptable salt thereof to a subject in need thereof.

In one aspect, the present disclosure relates to the use of a compound of formula (I) or a pharmaceutically acceptable salt thereof in the manufacture of a medicament for treating or preventing a cancer or a fibrosis-related disease.

In one aspect, the present disclosure relates to a compound of formula (I) or a pharmaceutically acceptable salt thereof for use in the treatment or prevention of a cancer or a fibrosis-related disease.

### Description of the invention

In one aspect, the present disclosure relates to compounds of formula (I): 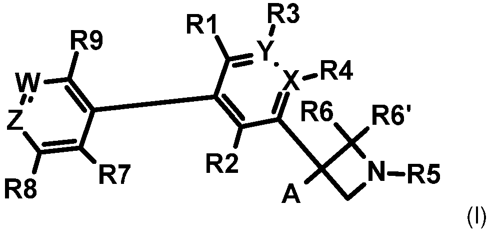 or a pharmaceutically acceptable salt, enantiomers, diastereomers, tautomers, solvates, isotope substituents, polymorphs, prodrugs or metabolites thereof, wherein:
A is a phenyl optionally substituted with 1 to 3 substituents selected from halogen, (C₁-C₆)alkyl and (C₁-C₆)alkoxy; or a 4- to 7-membered unsaturated monocyclic group comprising from 1 to 3 heteroatoms, preferably 1 or 2 heteroatoms, chosen from nitrogen, oxygen and sulfur;
W and Z are each independently CR₁₅ or N;
X and Y are each independently C or N;
R₁ is H, halogen, (C₁-C₆)alkyl or (C₁-C₆)alkoxy;
R₂ is H, halogen, (C₁-C₆)alkyl or (C₁-C₆)alkoxy;
R₃ is absent when Y is N, or R₃ is H, halogen, -OH, -COCH₃, (C₁-C₆)alkyl or (C₁-C₆)alkoxy when Y is C;
R₄ is absent when X is N, or R₄ is H, halogen, -OH, -COCH₃, (C₁-C₆)alkyl or (C₁-C₆)alkoxy when X is C;
R₅ is H, (C₁-C₆)alkyl optionally substituted with halogen, -OH, -CONHR₁₂, -CONH₂, - NR₁₂R₁₃, (C₁-C₆)alkoxy or (C₃-C₆)cycloalkyl; or (C₃-C₆)cycloalkyl optionally substituted with - OR₁₂, -CONHR₁₂, -CONH₂, -NR₁₂R₁₃;
R₆ and R₆' are each independently H, (C₁-C₆)alkyl or (C₃-C₆)cycloalkyl;
Or R₆ and R₆' are bond together to form a 3 or 4 membered carbocyclic ring;
R₇ is H, halogen, (C₁-C₆)alkyl or (C₁-C₆)alkoxy;
R₈ is H, halogen, (C₁-C₆)alkyl, (C₁-C₆)alkoxy, (C₁-C₆)alkoxy optionally substituted with (C₁-C₆)alkoxy, -OR₁₀, -OCH₂R₁₀ ; -NHR₁₀ ; -N(CH₃)R₁₀; or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₉ is (C₁-C₆)alkyl , (C₁-C₆)alkoxy, -CONHR₁₁, -C(NH)NHOH; -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₁ is H, -OH, -NH₂,(C₁-C₆)alkyl, (C₁-C₆)alkoxy, and -NHR₁₄ ;
R₁₂ is H or (C₁-C₆)alkyl;
R₁₃ is H or (C₁-C₆)alkyl;
R₁₄ is (C₁-C₆)alkyl;
R₁₅ is H, halogen, (C₁-C₆)alkyl or (C₁-C₆)alkoxy.

Any combination of the groups described above for the various variables is contemplated herein. Throughout the specification, groups and substituents thereof are chosen by one skilled in the field to provide stable moieties and compounds.

As used herein, the term "alkyl" means a linear or branched hydrocarbon moiety having the mentioned number of carbon atoms. In some embodiments, an alkyl comprises one to six carbon atoms (e.g., C₁-C₆ alkyl). In some embodiments, an alkyl comprises one to four carbon atoms (e.g., C₁-C₄ alkyl). In some embodiments, the alkyl group is selected from methyl, ethyl, propyl, 2-propyl, butyl, 2-methylpropyl, 2-butyl, tert-butyl, pentyl, 1,1-dimethylpropyl, 2,2-dimethylpropyl, 3-methylbutyl, 1-methylbutyl, 1-ethylpropyl, 1,2-dimethylpropyl, 1-methylethyl (isopropyl), 1-butyl (n-butyl), 1-methylpropyl (sec-butyl), pentyl or hexyl.

As used herein, the term "alkoxy" means a group -O-alkyl where alkyl is as defined above. As used herein, the term "cycloalkyl" means a 3- to 6-membered hydrocarbon cycle, which can be bridged, including cyclopropyl, cyclobutyl, cyclopentyl and cyclohexyl.

As used herein, the term "halogen" means F, Cl, Br or I, preferably F, Br or Cl.

In some embodiments, A is a phenyl optionally substituted with 1 to 3 substituents selected from halogen, (C₁-C₆)alkyl and (C₁-C₆)alkoxy.

In some embodiments, A is a 4- to 7-membered unsaturated monocyclic group comprising from 1 to 3 heteroatoms, preferably 1 or 2 heteroatoms, chosen from nitrogen, oxygen and sulfur. Examples of such monocyclic groups include the groups pyrrolyl, pyrazolyl, imidazolyl, oxazolyl, isoxazolyl, triazolyl, oxadiazolyl, furanyl, thienyl, thiazolyl, isothiazolyl, thiadiazolyl, pyridyl, pyridazinyl, pyrimidinyl, pyrazinyl, and triazinyl.

In some embodiments, A is a phenyl optionally substituted with 1 to 3 substituents selected from halogen, (C₁-C₆)alkyl and (C₁-C₆)alkoxy.

In some embodiments, W and Z are each CH.

In some embodiments, X and Y are each C.

In some embodiments, R₁ is halogen.

In some embodiments, R₂ is H, halogen or (C₁-C₆)alkyl.

In some embodiments, R₃ when present is H or halogen.

In some embodiments, R₄ when present is H.

In some embodiments, R₆ and R'₆ are each H.

In some embodiments, R₅ is H or (C₁-C₆)alkyl.

In some embodiments, R₇ is halogen or (C₁-C₆)alkyl.

In some embodiments, R₈ is H or (C₁-C₆)alkoxy optionally substituted with (C₁-C₆)alkoxy.

In some embodiments, R₁₁ is H or (C₁-C₆)alkyl.

In some embodiments, the compound of formula (I) is selected from:
2-[2-chloro-5-(3-phenylazetidin-3-yl)phenyl]-3-fluoro-4-(2-methoxyethoxy)benzamide;
2-[2-chloro-5-(3-phenylazetidin-3-yl)phenyl]-3-methyl-benzamide;
rel-(2aR)-2-[6-chloro-2-methyl-3-(3-phenylazetidin-3-yl)phenyl]-3-fluoro-4-(2-methoxyethoxy)benzamide;
rel-(2aS)-2-[6-chloro-2-methyl-3-(3-phenylazetidin-3-yl)phenyl]-3-fluoro-4-(2-methoxyethoxy)benzamide;
rel-(2aR)-2-[6-chloro-2-fluoro-3-(3-phenylazetidin-3-yl)phenyl]-3-fluoro-4-(2-methoxyethoxy)benzamide;
rel-(2aS)-2-[6-chloro-2-fluoro-3-(3-phenylazetidin-3-yl)phenyl]-3-fluoro-4-(2-methoxyethoxy)benzamide;
2-[2-chloro-5-(1-methyl-3-phenyl-azetidin-3-yl)phenyl]-3-fluoro-4-(2-methoxyethoxy)benzamide;
rel-(2aR)-2-[2,3-dichloro-6-fluoro-5-(3-phenylazetidin-3-yl)phenyl]-3-fluoro-4-(2-methoxyethoxy)benzamide;
rel-(2aS)-2-[2,3-dichloro-6-fluoro-5-(3-phenylazetidin-3-yl)phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

In some embodiments, the compound of formula (I) is selected from:
2-[2-chloro-5-(3-phenylazetidin-3-yl)phenyl]-3-fluoro-4-(2-methoxyethoxy)benzamide;
rel-(2aS)-2-[6-chloro-2-methyl-3-(3-phenylazetidin-3-yl)phenyl]-3-fluoro-4-(2-methoxyethoxy)benzamide;
rel-(2aR)-2-[6-chloro-2-fluoro-3-(3-phenylazetidin-3-yl)phenyl]-3-fluoro-4-(2-methoxyethoxy)benzamide;
2-[2-chloro-5-(1-methyl-3-phenyl-azetidin-3-yl)phenyl]-3-fluoro-4-(2-methoxyethoxy)benzamide;
rel-(2aS)-2-[2,3-dichloro-6-fluoro-5-(3-phenylazetidin-3-yl)phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

In the context of the present disclosure, the various embodiments described herein can be combined.

Pharmaceutically acceptable salts of the compounds of formula (I) include those disclosed in the Handbook of Pharmaceutical Salts: Properties, Selection and Use (P. H. Stahl and C. G. Wermuth, Weinheim/Zürich:Wiley-VCH/VHCA, 2002).

In one aspect, the present disclosure relates to enantiomers, diastereomers, tautomers, solvates, isotope substituents, polymorphs, prodrugs or metabolites of the compounds of formula (I).

As used herein, the term "prodrug" is meant to indicate a compound that is converted under physiological conditions or by solvolysis to a biologically active compound described herein. Thus, the term "prodrug" refers to a precursor of a biologically active compound that is pharmaceutically acceptable. In some embodiments, a prodrug is inactive when administered to a subject, but is converted *in vivo* to an active compound, for example, by hydrolysis. The prodrug compound often offers advantages of solubility, tissue compatibility or delayed release in a mammalian organism. The term "prodrug" is also meant to include any covalently bonded carriers, which release the active compound *in vivo* when such prodrug is administered to a mammalian subject. In some embodiments, prodrugs of an active compound, as described herein, are prepared by modifying functional groups present in the active compound in such a way that the modifications are cleaved, either in routine manipulation or *in vivo,* to the parent active compound.

As used herein, the term "solvate" refers to an aggregate of molecules comprising one or more compounds of the present disclosure and one or more solvent molecules. The solvent may be water, in which case the solvate may be a hydrate. Alternatively, the solvent may be an organic solvent. Thus, the compounds of the present disclosure may exist as hydrates, including monohydrate, dihydrate, hemihydrate, sesquihydrate, trihydrate, tetrahydrate, and the like, and as corresponding solvated forms. The disclosed compounds may be true solvates, while in other cases, the disclosed compounds may only remain in water or a mixture of water plus some solvent.

The compounds disclosed herein, or pharmaceutically acceptable salts thereof, may contain one or more chiral carbon atoms, and thus may yield enantiomers, diastereomers, and other stereoisomeric forms. Each chiral carbon atom may be defined as (R)- or (S)- based on stereochemistry. Unless stated otherwise, it is intended that all stereoisomeric forms of the compounds disclosed herein are contemplated by this disclosure. When the compounds described herein contain alkene double bonds, and unless specified otherwise, it is intended that this disclosure includes both E and Z geometric isomers (e.g., cis or trans). Likewise, all possible isomers, as well as their racemic and optically pure forms, and all tautomeric forms are also intended to be included. The term "geometric isomer" refers to E or Z geometric isomers (e.g., cis or trans) of an alkene double bond. The disclosure is intended to include all possible isomers, as well as racemates and optically pure forms thereof. The preparation of the compounds disclosed herein may select racemates, diastereomers or enantiomers as starting materials or intermediates. Optically active isomers may be prepared using chiral synthesizers or chiral reagents, or resolved using conventional techniques, such as crystallization and chiral chromatography.

Conventional techniques for preparing/separating individual isomers include chiral synthesis from suitable optically pure precursors or using chiral reagents/catalysts, separation of racemates/diastereoisomers (or racemates/diastereoisomers of salts or derivatives) using, for example, chiral high-performance liquid chromatography, chiral Supercritical Fluid chromatography (SFC) or recrystallization

As used herein, the term "stereoisomer" refers to a compound consisting of the same atom, bonded by the same bond, but having a different three-dimensional structure. The present disclosure will cover various stereoisomers and mixtures thereof.

As used herein, the term "tautomer" refers to a molecule wherein a proton shift from one atom of a molecule to another atom of the same molecule is possible. The compounds presented herein, in certain embodiments, exist as tautomers. In circumstances where tautomerization is possible, a chemical equilibrium of the tautomers will exist. The exact ratio of the tautomers depends on several factors, including physical state, temperature, solvent, and pH. All tautomeric forms of the compounds disclosed herein will also be included within the scope of the present disclosure.

The present disclosure also encompasses all suitable isotopic substitutions (or isotopic variations) of the compounds of the present disclosure, or pharmaceutically acceptable salts thereof. Isotopic variations of the compounds disclosed herein, or pharmaceutically acceptable salts thereof, are defined as those in which at least one atom is replaced by an atom having the same number of atoms but different in atomic mass from that found frequently in nature. Isotopes that may be incorporated into the compounds of the present disclosure and pharmaceutically acceptable salts thereof include, but are not limited to, isotopes of H, C, N, and O, such as ²H, ³H, ¹¹C, ¹³C, ¹⁴C, ¹⁵N, ¹⁷O, ¹⁸O, ³⁵S, ¹⁸F, ³⁶Cl, and ¹²⁵I. Isotopic variations of the compounds described herein, may be prepared by conventional techniques using suitable isotopic variations of suitable reagents.

As used herein, the terms "crystalline form" and "polymorph" are used interchangeably and refer to crystalline structures in which a compound (or a salt, enantiomer, diastereomer, tautomer, solvate, isotope substituent, polymorph, prodrug or metabolite) can crystallize in different crystal stacking arrangements (all having the same elemental composition). Different crystal forms generally have different X-ray diffraction patterns, infrared spectra, melting points, density hardness, crystal shape, optical and electrical properties, stability and solubility. Recrystallization solvents, crystallization rates, storage temperatures, or other factors may lead to dominance of one of the crystal forms. Polymorphs of compounds can be prepared by crystallization under different conditions.

As used herein, the term "metabolite" refers to derivatives that are also pharmacologically active resulting from the metabolic process of the parent pharmaceutical active ingredient in a subject, for example, such metabolites may result from oxidation, reduction, hydrolysis, amidation, deamidation, esterification, enzymatic cleavage, and the like of the administered compound or salt or prodrug.

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use as a medicament.

The compounds of formula (I) can be administered in the form of a pharmaceutical composition. Accordingly, in one aspect, the present disclosure relates to a pharmaceutical composition comprising a compound of formula (I) or one of its pharmaceutically acceptable salts, enantiomer, diastereomer, tautomer, solvate, isotope substituent, polymorph, prodrug or metabolite thereof. In some embodiments, the compound of formula (I) as described herein is substantially pure, in that it contains less than about 5%, or less than about 1%, or less than about 0.1%, of other organic small molecules, such as contaminating intermediates or by-products that are created, for example, in one or more of the steps of a synthesis method. Administration *in vivo* may be by any routes, including oral, parenteral, topical, buccal, parenteral (e.g. intramuscular, subcutaneous, intradermal, or intravenous), intranasal, sublingual, intratracheal, inhalation, ocular, vaginal and rectal routes. The most suitable form of administration in any given case will depend on the degree and severity of the condition being treated and on the nature of the particular compound being used. For example, disclosed compositions are formulated as a unit dose, and/or are formulated for oral or subcutaneous administration. Depending on the intended mode of *in vivo* administration, said composition may be in a solid dosage form, a semi-solid dosage form or a liquid dosage form, the list being not limitative. In some embodiments, the pharmaceutical composition is a solid dosage form. Exemplary solid dosage forms include tablets, capsules, stick-packs, dragees, sachets, lozenges, powders, pills, or granules. Preferred solid dosage forms include tablets, capsules and stick-packs, tablets being especially preferred.

In some embodiments, the pharmaceutical composition is a liquid dosage form. Exemplary liquid dosage forms include emulsions, microemulsions, solutions, suspensions, syrups and elixirs.

Advantageously, the compositions are administered in unit dosage forms suitable for single administration of precise dosage amounts.

The pharmaceutical compositions may also include, depending on the formulation desired, one or more pharmaceutically acceptable excipient(s). The choice of excipient(s) will to a large extent depend on factors such as the particular mode of administration, the effect of the excipient on solubility and stability, and the nature of the dosage form. The pharmaceutical compositions of the invention can be prepared by conventional methods, as described e.g. in Remington's Pharmaceutical Sciences, 19th Edition (Mack Publishing Company, 1995), incorporated herein by reference.

In some embodiments, the pharmaceutical composition comprises from 0.01 mg to 1000 mg of compound of formula (I) or a pharmaceutically acceptable salt, enantiomer, diastereomer, tautomer, solvate, isotope substituent, polymorph, prodrug or metabolite thereof.

In some embodiments, the pharmaceutical composition further comprises at least one other active pharmaceutical ingredient or its derivatives, prodrugs, solvates, tautomers or stereoisomers thereof as well as the pharmaceutically acceptable salts of each of the foregoing, including mixtures thereof in all ratios, wherein the at least one other active pharmaceutical ingredient is other than a compound of formula (I); preferably, at least one other active pharmaceutical ingredient that is useful in the treatment, prevention, suppression and/or amelioration of medicinal conditions or pathologies for which the compounds of the present disclosure are useful.

In some embodiments, the at least one other active pharmaceutical ingredient is selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

As used herein, the term "chemotherapeutic agent" includes, for example, alkylating agents such as nitrogen mustards, alkylsulfonates, nitrosoureas, triazines, ethylenimines and platinum derivatives; antimetabolites such as 5-fluorouracil (5-FU), 6-mercaptopurine (6-MP), cytarabine, capecitabine, fludarabine, gemcitabine, methotrexate, pemetrexed, pentostatin and thioguanine; anthracyclines, such as doxorubicin, daunorubicin, idarubicin and epirubicin; topoisomerase inhibitors, such as topotecan, irinotecan, etoposide and teniposide; mitotic spindle inhibitors, such as plant alkaloids and taxanes; and hormonal agents, such as corticosteroid hormones and sex hormones, the list of chemotherapeutic agent being not limitative.

Non-limiting examples of nitrogen mustards are chlorambucil, cyclophosphamide, ifosfamide, and melphalan. Non-limiting example of alkylsulfonates is busulfan. Non-limiting examples of nitrosoureas are streptozotocin, carmustine, and lomustine. Non-limiting example of triazines is dacarbazine. Non-limiting examples of ethylenimines are thiotepa and altretamine. Non-limiting examples of platinum derivatives are cisplatin, carboplatin and oxaliplatin. Non-limiting examples of plant alkaloids are vinblastine, vincristine and vinorelbine. Non-limiting examples of taxanes are paclitaxel and docetaxel. Non-limiting examples of corticosteroid hormones are prednisone, methylprednisolone and dexamethasone. Non-limiting examples of sex hormones are tamoxifen and leuprolide.

As used herein, "radiotherapeutic treatment" may consist of gamma-radiation, X-ray radiation, electrons or photons, external radiotherapy or internal radiotherapy. As used herein, the term "radiotherapeutic treatment", is also intended to refer to any radiotherapeutic treatment known to one of skill in the art to be effective to treat or ameliorate cancer, without limitation. For instance, the radiotherapeutic treatment can be an agent such as those administered in brachytherapy or radionuclide therapy. Such methods can optionally further comprise the administration of one or more additional cancer therapies, such as, but not limited to, chemotherapies. For instance, the radiotherapeutic treatment can include radioligand therapy (vectorized internal radiotherapy), such as for example but without limitations lutecium-177 (Lu177), vipivotide tetraxetan, andibritumomab tiuxetan.

Examples of immunotherapy agents include without limitations monoclonal antibodies, antibody-drug conjugates, bispecific antibodies, immunoconjugates, checkpoint modulators, cytokines and photoimmunotherapy.

Examples of immunotherapy drug based on oncolytic viruses include without limitations T-VEC (Imlygic^{®}), and other oncolityc virus such as for example: adenovirus, herpes simplex virus, maraba virus, measles, newcastle disease virus, picornavirus, reovirus, vaccinia virus and vesicular stomatitis virus.

Such combination of two or more active pharmaceutical ingredients may be safer or more effective than either compound of formula (I) alone, or the combination is safer or more effective than it would be expected based on the additive properties of the individual drugs (at least one other active pharmaceutical ingredient and compound of formula (I)). Such at least one other active pharmaceutical ingredient may be administered, by a route and in an amount commonly used contemporaneously or sequentially with a compound of formula (I) of the present disclosure. When a compound of formula (I) of the present disclosure is used contemporaneously with at least one other active pharmaceutical ingredient, a combination product containing such at least one other active pharmaceutical ingredient and the compound of formula (I) of the present disclosure - also referred to as "fixed dose combination" - is preferred. However, combination therapy also includes therapies in which the compound of formula (I) of the present disclosure and at least one other active pharmaceutical ingredient are administered on different overlapping schedules. It is contemplated that when used in combination with at least one other active pharmaceutical ingredient, the compound of formula (I) of the present disclosure or the at least one other active pharmaceutical ingredient or both may be used effectively in lower doses than when each is used alone. Accordingly, the pharmaceutical compositions of the present invention include those that contain at least one other active pharmaceutical ingredient, in addition to a compound of formula (I) of the invention.

Consequently, in one aspect, the present disclosure relates to a pharmaceutical composition which comprises a compound of formula (I) according to the invention or a pharmaceutically acceptable salt, enantiomer, diastereomer, tautomer, solvate, isotope substituent, polymorph, prodrug or metabolite thereof, and at least one of the active pharmaceutical ingredients described above as combination partners, optionally together with one or more pharmaceutically acceptable excipients.

The compounds of formula (I) of the invention or pharmaceutically acceptable salts, enantiomers, diastereomers, tautomers, solvates, isotope substituents, polymorphs, prodrugs or metabolite thereof, and the additional active pharmaceutical ingredient(s) to be combined therewith may both be present together in one formulation, for example a tablet or capsule, or separately in two identical or different formulations, for example as so-called kit-of-parts.

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use for the treatment or prevention of a cancer.

In one aspect, the present disclosure relates to the use of a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a cancer.

In one aspect, the present disclosure relates to a method of treating or preventing a cancer in a patient, comprising administering to the patient, a therapeutically effective amount of a compound of formula (I) as defined above, or a pharmaceutically acceptable salt, enantiomer, diastereomer, tautomer, solvate, isotope substituent, polymorph, prodrug or metabolite thereof.

In one aspect, the present disclosure relates to a pharmaceutical composition comprising a compound of formula (I) as defined above, or a pharmaceutically acceptable salt, enantiomer, diastereomer, tautomer, solvate, isotope substituent, polymorph, prodrug or metabolite thereof, for use for the treatment or prevention of a cancer.

In one aspect, the present disclosure relates to the use of a pharmaceutical composition comprising a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a cancer.

In one aspect, the present disclosure relates to a method of treating or preventing a cancer in a patient, comprising administering to the patient a pharmaceutical composition comprising a therapeutically effective amount of a compound of formula (I) as defined above, or a pharmaceutically acceptable salt, enantiomer, diastereomer, tautomer, solvate, isotope substituent, polymorph, prodrug or metabolite thereof.

In some embodiments, the cancer is a solid tumor. In some embodiments, the cancer is a liquid tumor. In some embodiments, the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.

In some embodiments, the pharmaceutical composition useful for the treatment or prevention of a cancer further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

In embodiments that involve administering to a patient a compound of formula (I) as defined above, or a pharmaceutically acceptable salt, enantiomer, diastereomer, tautomer, solvate, isotope substituent, polymorph, prodrug or metabolite thereof, the expressions "effective amount", "therapeutically effective amount", "amount effective to treat" or "pharmaceutically effective amount" denote the amount of compound of formula (I) as defined above, or a pharmaceutically acceptable salt, enantiomer, diastereomer, tautomer, solvate, isotope substituent, polymorph, prodrug or metabolite thereof, which is needed to inhibit or reverse a disease condition (e.g., to treat cancer or fibrosis related diseases). Determining an effective amount specifically depends on such factors as toxicity and efficacy of the medicament. These factors will differ depending on other factors such as potency, relative bioavailability, patient body weight, severity of adverse side-effects and preferred mode of administration. Toxicity may be determined using methods well known in the art. Efficacy may be determined utilizing the same guidance. An effective amount, therefore, is an amount that is deemed by the clinician to be toxicologically tolerable, yet efficacious. This amount can be a fixed dose for all subjects being treated, or can vary depending upon the physical condition of the subject to be treated, a professional assessment of the medical situation, and other relevant factors.

As used herein, the term "treatment" or "treating" refers to any process, action, application, therapy, or the like, wherein the patient is under aid, in particular, medical, or veterinarian aid with the object of improving the patient's condition, in particular leading to a cure or a treatment which alleviates, improves and/or eliminates, reduces and/or stabilizes the symptoms of a disease or the suffering that it causes, either directly or indirectly.

As used herein, the term "prevent", "prevention" or "prophylaxis" or "preventive treatment" or "prophylactic treatment" includes a treatment leading to the prevention of a disease as well as a treatment reducing and/or delaying the incidence of a disease or the risk of the disease occurring.

As used herein, "patient" or "subject" refers to a human individual or an animal different from a human. The patient is for example a human or an animal liable to have cancer and/or fibrosis related disease or suffering from such diseases. The patient is advantageously a human being. The patient may be a child (human patient aged 18 or under) or an adult (human patient over 18). In another embodiment, the subject is a non-human animal including, but are not limited to dog, cat, guinea pig, rabbit, rat, mouse, horse, cattle, bear, cow, ape, monkey, orangutan, and chimpanzee, and so on. In the context of the present disclosure the terms "patient" and "subject" are used interchangeably

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use for the treatment or prevention of a fibrosis related disease.

In one aspect, the present disclosure relates to the use of a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a fibrosis related disease.

In one aspect, the present disclosure relates to a method of treating or preventing a fibrosis related disease in a patient, comprising administering to the patient an effective amount of a compound of formula (I) as defined above, or a pharmaceutically acceptable salt, enantiomer, diastereomer, tautomer, solvate, isotope substituent, polymorph, prodrug or metabolite thereof.

In one aspect the present disclosure relates to a pharmaceutical composition comprising a compound of formula (I) as defined above, or a pharmaceutically acceptable salt, enantiomer, diastereomer, tautomer, solvate, isotope substituent, polymorph, prodrug or metabolite thereof, for use for the treatment or prevention of a fibrosis related disease.

In one aspect, the present disclosure relates to the use of a pharmaceutical composition comprising a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a fibrosis related disease.

In one aspect, the present disclosure relates to a method of treating or preventing a fibrosis related disease in a patient, comprising administering to the patient a pharmaceutical composition comprising an effective amount of a compound of formula (I) as defined above, or a pharmaceutically acceptable salt, enantiomer, diastereomer, tautomer, solvate, isotope substituent, polymorph, prodrug or metabolite thereof.

In some embodiments, fibrosis related diseases include, without limitations, skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease).

In some embodiments, the doses of the composition comprising at least one compound of formula (I) as described herein differ, depending upon the patient's (e.g., human) condition, that is, stage of the disease, general health status, age, and other factors that a person skilled in the medical art will use to determine dose. In some instances, pharmaceutical compositions are administered in a manner appropriate to the disease to be treated as determined by skilled practitioner. An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity) Optimal doses are generally determined using experimental models and/or clinical trials. In some embodiments, the optimal dose depends upon the body mass, weight, or blood volume of the patient and on the activity of the compound of formula (I) - or pharmaceutically acceptable salt, enantiomer, diastereomer, tautomer, solvate, isotope substituent, polymorph, prodrug or metabolite thereof.

In one aspect, the present disclosure provides a method of modulating one or more of proteins encompassed by, or related to, the Hippo pathway in a subject, comprising administering to a subject in need thereof a compound of formula (I), or a pharmaceutically acceptable salt thereof.

In some embodiments, a modulator of the Hippo pathway is a modulator of a core protein of the Hippo pathway. In some embodiments, a modulator of the Hippo pathway is a modulator of YAP. In some embodiments, a modulator of the Hippo pathway is a modulator of TAZ. In some embodiments, a modulator of the Hippo pathway is a modulator of TEAD. In some embodiments, the compound of formula (I) is a modulator of the Hippo pathway, in particular a modulator of a core protein of the Hippo pathway. In some embodiments, the compound of formula (I) is a modulator of YAP. In some embodiments, the compound of formula (I) is a modulator of TAZ. In some embodiments, the compound of formula (I) is a modulator of TEAD.

In one aspect, the present disclosure provides a method of inhibiting one or more of proteins encompassed by, or related to, the Hippo pathway in a subject, comprising administering to a subject in need thereof a compound of formula (I), or a pharmaceutically acceptable salt thereof.

In some embodiments, an inhibitor of the Hippo pathway is an inhibitor of a core protein of the Hippo pathway. In some embodiments, an inhibitor of the Hippo pathway is an inhibitor of YAP. In some embodiments, an inhibitor of the Hippo pathway is an inhibitor of TAZ. In some embodiments, an inhibitor of the Hippo pathway is an inhibitor of TEAD. In some embodiments, the compound of formula (I) is an inhibitor of the Hippo pathway, in particular an inhibitor of a core protein of the Hippo pathway. In some embodiments, the compound of formula (I) is an inhibitor of YAP. In some embodiments, the compound of formula (I) is an inhibitor of TAZ. In some embodiments, the compound of formula (I) is an inhibitor of TEAD.

In one aspect, the present disclosure provides a method of activating one or more of proteins encompassed by, or related to, the Hippo pathway in a subject, comprising administering to a subject in need thereof a compound of formula (I), or a pharmaceutically acceptable salt thereof.

In some embodiments, an activator of the Hippo pathway is an activator of a core protein of the Hippo pathway. In some embodiments, an activator of the Hippo pathway is an activator of YAP. In some embodiments, an activator of the Hippo pathway is an activator of TAZ. In some embodiments, an activator of the Hippo pathway is an activator of TEAD. In some embodiments, the compound of formula (I) is an activator of the Hippo pathway, in particular an activator of a core protein of the Hippo pathway. In some embodiments, the compound of formula (I) is an activator of YAP. In some embodiments, the compound of formula (I) is an activator of TAZ. In some embodiments, the compound of formula (I) is an activator of TEAD.

In one aspect, provided herein are compound of formula (I) as disclosed herein or a pharmaceutically acceptable salt thereof, or the pharmaceutical composition thereof as described herein, for treating or preventing a disease or disorder by modulating or inhibiting or activating one or more of proteins encompassed by, or related to, the Hippo pathway, in a subject in need thereof. In yet another aspect, provided herein are uses of a compound of formula (I) as disclosed herein or a pharmaceutically acceptable salt thereof, or the pharmaceutical composition thereof as described herein, in the preparation of a medicament for treating or preventing a disease or disorder by modulating or inhibiting or activating one or more of proteins encompassed by, or related to, the Hippo pathway, in a subject in need thereof.

In some embodiments, a modulator of the Hippo pathway is a modulator of a core protein of the Hippo pathway. In some embodiments, a modulator of the Hippo pathway is a modulator of YAP. In some embodiments, a modulator of the Hippo pathway is a modulator of TAZ. In some embodiments, a modulator of the Hippo pathway is a modulator of TEAD. In some embodiments, the compound of formula (I) is a modulator of the Hippo pathway, in particular a modulator of a core protein of the Hippo pathway. In some embodiments, the compound of formula (I) is a modulator of YAP. In some embodiments, the compound of formula (I) is a modulator of TAZ. In some embodiments, the compound of formula (I) is a modulator of TEAD.

In some embodiments, an inhibitor of the Hippo pathway is an inhibitor of a core protein of the Hippo pathway. In some embodiments, an inhibitor of the Hippo pathway is an inhibitor of YAP. In some embodiments, an inhibitor of the Hippo pathway is an inhibitor of TAZ. In some embodiments, an inhibitor of the Hippo pathway is an inhibitor of TEAD. In some embodiments, the compound of formula (I) is an inhibitor of the Hippo pathway, in particular an inhibitor of a core protein of the Hippo pathway. In some embodiments, the compound of formula (I) is an inhibitor of YAP. In some embodiments, the compound of formula (I) is an inhibitor of TAZ. In some embodiments, the compound of formula (I) is an inhibitor of TEAD. In some embodiments, an activator of the Hippo pathway is an activator of a core protein of the Hippo pathway. In some embodiments, an activator of the Hippo pathway is an activator of YAP. In some embodiments, an activator of the Hippo pathway is an activator of TAZ. In some embodiments, an activator of the Hippo pathway is an activator of TEAD. In some embodiments, the compound of formula (I) is an activator of the Hippo pathway, in particular an activator of a core protein of the Hippo pathway. In some embodiments, the compound of formula (I) is an activator of YAP. In some embodiments, the compound of formula (I) is an activator of TAZ. In some embodiments, the compound of formula (I) is an activator of TEAD.

### General synthetic schemes

The compounds of formula (I) can be prepared using the synthetic transformations illustrated in schemes I and II. Starting materials are either commercially available or can be prepared by procedures described herein (schemes III, IV), by literature procedures, or by procedures well known to one skilled in the art of organic chemistry. All general synthetic schemes are illustrated with A = phenyl.

In scheme I, the coupling reaction, starting from the bromo derivative (prepared following scheme III), can be performed by a Suzuki or Stille reaction using the protected azetidine derivative (step a). The Suzuki coupling can be performed with the corresponding boronate or boronic acid (commercially available or prepared following scheme IV) using classical palladium catalysts (such as Pd(dppf)Cl₂, XPHOS PD G2/RUPHOS, APHOS PD G3, Pd₂dba₃/N-XANTPHOS, etc.) in the presence of a base (such as potassium carbonate, tri-potassium phosphate, etc.). The Stille coupling can be performed with the corresponding stannyl derivative (commercially available or prepared following scheme IV) using classical palladium catalysts (such as Pd(PtBu₃)₂). In step b, a Friedel Craft reaction is performed using aluminum chloride as catalyst. In step c, in case of R18 is nitrile, hydrolysis is done using Ghaffar-Parkins reagent to give the corresponding amide group. 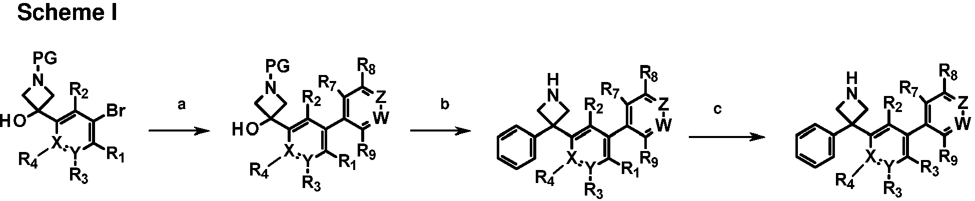 (PG = protecting group)

In scheme II, step a, a Friedel Craft reaction is performed using aluminum chloride as catalyst. In scheme b, the N-alkylate product is obtained either by alkylation with the corresponding halogeno reactant in the presence of a base (such as NaH, potassium carbonate, cesium carbonate...) or by reductive amination with the corresponding aldehyde/ketone in the presence of a reducer (such as NaBH₄, NaBH(OAc)₃......). In step c, the coupling reaction can be performed by a Suzuki or Stille reaction. The Suzuki coupling can be performed with the corresponding boronate or boronic acid (commercially available or prepared following scheme IV) using classical palladium catalysts (such as Pd(dppf)Cl₂, XPHOS PD G2/RUPHOS, APHOS PD G3, Pd₂dba₃/N-XANTPHOS......) in the presence of a base (such as potassium carbonate, tri-potassium phosphate...). The Stille coupling can be performed with the corresponding stannyl derivative (commercially available or prepared following scheme IV) using classical palladium catalysts (such as Pd(PtBu₃)₂.....). In step d, when R₉ is nitrile, the hydrolysis is done using Ghaffar-Parkins reagent to give the corresponding amide group.

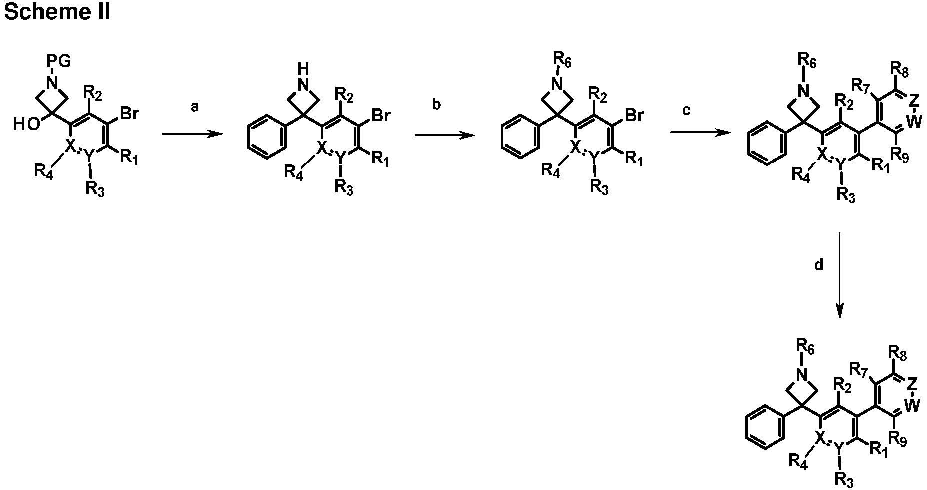

In Scheme III, step a, the compound can be prepared using either a Grignard reaction or by lithiation. The Grignard reagent is prepared starting from the corresponding aryl-iodide (either commercially available or prepared following protocols described in the literature) with an isopropylmagnesium chloride lithium chloride complex and then reacted with the protected azetidinone. The lithiation is performed with a lithium species (such as nBuLi, tBuLi....).

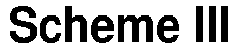

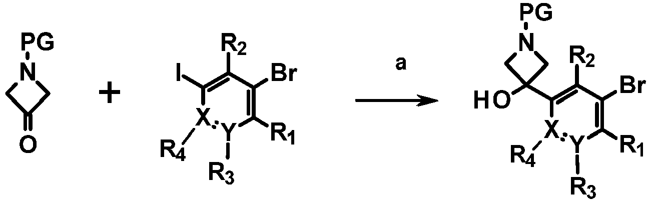

Scheme IV describe a general procedure for the preparation of the synthons used in the Suzuki or Stille couplings. In step a, the boronate derivative is prepared with bis(pinacolato)diboron, using Pd(dppf)Cl₂ as catalyst, in the presence of potassium acetate. In step b, the Stille reactant is formed with trimethyl stannyl chloride using n-butyl-lithium as base.

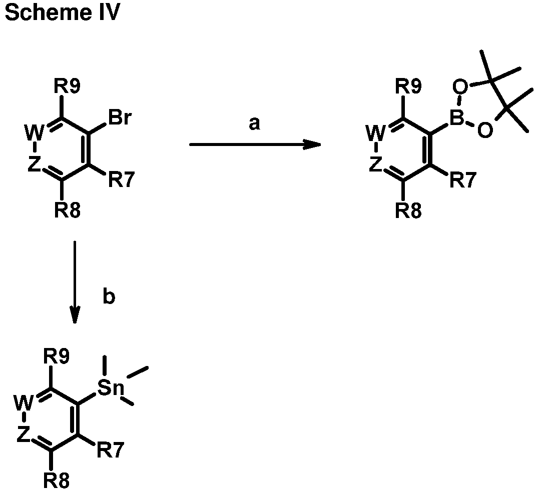

### EXPERIMENTAL PROCEDURES

### ABBREVIATIONS

AcOH = Acetic acid; ACN = Acetonitrile; APHOS PD G3 = [4-(Di-tert-butylphosphino)-N,N-dimethylaniline-2-(2'-aminobiphenyl)]palladium(II) methanesulfonate; br, = broad; n-BuLi = n-Butyllithium; t-BuLi = tert-Butyllitium; CDCl₃ = Chloroform deutered; CV = column volume; d = Doublet; DCM = Dichloromethane; dd = Doublet of doublets; DIPEA = Diisopropylethylamine; DMF = N,N-Dimethylformamide; DMSO = Dimethyl-sulfoxide; eq = Equivalent(s); EtOAc = Ethyl acetate; Et₂O = Diethyl-Ether; EtOH = Ethanol; g = Gram(s); h = Hour(s); HCl = Hydrochloric acid; Hz = Hertz; LC/MS = Liquid chromatography/mass spectrometry; m = Multiplet; min = Minute; MeOH = Methanol; mg = Milligram; MgSO₄ = Magnesium sulfate; min = Minute(s); mL = milliliter; µL = microliter, mmol = Millimole; mp = Melting point; MtBE = methyl tert-butyl ether; MW = Microwave; NaH = Sodium hydride; NaHCO₃ = Sodium bicarbonate; NaBH₄ = Sodium borohydride; NaBH(OAc)₃ = Sodium triacetoxyborohydride;;Na₂S₂O₃ = Sodium thiosulfate; Na₂SO₄ = Sodium sulfate; NCS = N-Chlorosuccinimide; NH₃ = Ammonia; NH₄Cl = Ammonium chloride; NMM = N-methyl morpholine; NMP = N-methylpyrrolidone; NMR = Nuclear magnetic resonance; PBS = Phosphate buffered saline; Pd(dppf)Cl₂ = Dichloro[1,1'-bis(diphenyl phosphino)ferrocene]palladium (II); Pd₂(dba)₃ = Bis(dibenzylidenacetone)palladium(0); Pd(PtBu₃)₂= Bis(tri-tert-butylphosphine)palladium(0); ppm = Parts per million; q = Quadruplet ; quant = Quantitative ; quint = Quintuplet ; Rt = Retention time ; rt = Room temperature; RUPHOS = 2-Dicyclohexylphosphino-2',6'-diisopropoxybiphenyl; s = singlet; sept = septuplet; sext = sextuplet; ; t = triplet; THF = Tetrahydrofuran; N-XANTPHOS = 4,6-Bis(diphenylphosphino)-10H-phenoxazine, 4,6-Bis(diphenylphosphino)phenoxazine; XPHOS PD G2 = Chloro(2-dicyclohexylphosphino-2',4',6'-triisopropyl-1,1'-biphenyl)[2-(2'-amino-1,1'-biphenyl)]-palladium(II).

### Analytical and semi-preparative Methods

| **Method** | **Description** |
|---|---|
| *Analytical A* | *Column: KINETEX XB-C18 core-shell (Dimensions: 30 x 3 mm, 2.6 µm)* |
| | *Column temperature: 45 °C* |
| | *Mobile Phase: A 1: Water (0.1% v*/*v AcOH) B1: ACN (0.1% v*/*v AcOH)* |
| | *Gradient: from 10% to 100% B1 within 3.15 min* |
| | *Flow rate: 1.4 ml*/*min, UV detection: DAD 210-260 nm, MS detection: ESI positive* |
| *Semi-preparative B* | *Column: Kinetex C18,30 x 150mm 5µm (phenomenex; 330mg),* |
| | *Flowrate : 42 ml*/*min,* |
| | *Mobile phase: H₂O with 0.1% formic acid* / *ACN + 0.1 % formic acid (% ACN : 0 to 0,5 min, 10% ; 0,5 to 1 min, 10 to 30% ; 1 to 8 min, 30 to 45%; 8 to 9 min,45 to 90%; 9 to 9,5 min, 90 à 100%)* |
| *Analytical C* | *Column : ACQUITY BEH Shield RP18* |
| | *Column temperature: 45 °C* |
| | *Mobile Phase: A: Water (0.1 % v*/*v AcOH) B: ACN (0.1 % v*/*v AcOH)* |
| | *Gradient: from 5% to 95% B within 2.5 min* |
| | *Flow rate: 0.8 ml*/*min, UV detection: DAD 210-260 nm, MS detection: ESI positive* |
| *Chiral D* | *Column : Chiralpak I8, 250 *4.6mm *5pm* |
| | *Column temperature: -4 °C* |
| | *Mobile phase : 97% Heptane* / *3% IPA +0.1% Ethanolamine* |
| | *Flowrate : 1ml*/*min* , *UV detection λ = 240nm* |
| *Analytical E* | *Column : Waters X bridge C18 50 x 2.1 mm x 3.5 µm* |
| | *Column temperature: 45 °C* |
| | *Mobile phase: A* = *H₂O + 0.1% NH₃ B* = *ACN + 0.1% NH₃* |
| | *Flowrate : 0.5ml*/*min* , *UV detection A = 210-260nm* |

### Example 1: 2-[2-chloro-5-(3-phenylazetidin-3-yl)phenyl]-3-fluoro-4-(2-methoxyethoxy)benzamide

### Step a: 2-Bromo-3-fluoro-4-(2-methoxyethoxy)benzonitrile

Under argon atmosphere 2-Methoxyethanol (3.0 mL, 37.8 mmol, 1.1 eq.) was dissolved in dry DMF (105.0 mL) and cooled to 0°C, then NaH (60% in mineral oil, 1.9 g, 48.2 mmol, 1.4 eq.) was added portion wise. After 5 min, 2-Bromo-3,4-difluorobenzonitrile (7.5 g, 34.4 mmol, 1.0 eq.) was added slowly (intense foam formation) and stirred at 0°C for 1.5 h. After completion the mixture was quenched with a saturated aqueous NH₄Cl solution then extracted with EtOAc. The organic layer was washed with a saturated NH₄Cl solution, dried and concentrated. The crude was purified by column chromatography (eluent: 0-35% EtOAc in cyclohexane) to obtain 2-Bromo-3-fluoro-4-(2-methoxyethoxy)benzonitrile (5.61 g 59.5%) as a pale-yellow solid. ¹H NMR (400 MHz, DMSO-*d₆*) δ ppm 7.80 (dd, *J*=8.80, 1.87 Hz, 1 H), 7.40 (dd, *J*=8.80, 8.03 Hz, 1 H), 4.33 (m, 2 H), 3.70 (m, 2 H), 3.31 (s, 3 H).

### Step b: 3-Fluoro-4-(2-methoxyethoxy)-2-(4,4,5,5-tetramethyl-1,3,2-dioxaborolan-2-yl)benzonitrile

Under Argon atmosphere 2-Bromo-3-fluoro-4-(2-methoxyethoxy)benzonitrile (3.61 g, 13.17 mmol, 1.0 eq.) was dissolved in dry Et₂O (132.0 mL) and cooled to -78°C. n-BuLi (2.5 M solution in hexane, 8.96 mL, 22.39 mmol, 1.7 eq.) was added slowly at -78°C and stirred at this temperature for 30 min. Then, 2-isopropoxy-4,4,5,5-tetramethyl-1,3,2-dioxaborolane (5.12 mL, 25.02 mmol, 1.9 eq.) was added and stirring was continued at -78°C for 3 h . After completion, the reaction mixture was quenched with 1 N aq. HCl solution while cooling with an ice-bath and extracted with EtOAc. The combined organic phases were washed with brine, dried and concentrated. The crude product was triturated with n-heptane (2-3x) and dried to obtain 3-Fluoro-4-(2-methoxyethoxy)-2-(4,4,5,5-tetramethyl-1,3,2-dioxaborolan-2-yl)benzonitrile (2.94 g, 69%) as a pale-yellow solid. ¹H NMR (400 MHz, DMSO-*d₆*) δ ppm 7.68 (dd, *J*=8.47, 0.99 Hz, 1 H), 7.42 (t, *J*=8.64 Hz, 1 H), 4.28 (m, 2 H), 3.68 (m, 2 H), 3.30 (s, 3 H), 1.33 (s, 12 H).

### Step c: tert-butyl 3-(3-bromo-4-chloro-phenyl)-3-hydroxy-azetidine-1-carboxylate

Under argon atmosphere, 1-bromo-2-chloro-5-iodobenzene (3.71 g, 11.7 mmol, 2 eq.) was solubilized in anhydrous THF (40 mL) and the solution was cooled to 0°C. Isopropylmagnesium chloride lithium chloride complex (9 mL, 12 mmol, 2 eq.) was added dropwise and the solution was stirred at 0°C for 45 min. This mixture was cooled to -70°C and cannulated to a solution of tert-butyl 3-oxoazetidine-1-carboxylate (1.00 g, 5.84 mmol, 1 eq.) in THF (40 mL) at -70°C (20 min of addition). The reaction mixture was stirred at -70°C for 1h then overnight at rt. The reaction mixture was quenched with a saturated solution of NH₄Cl and the aqueous layer was extracted with EtOAc (3x). The resulting organic layer was dried over MgSO₄, filtered and concentrated. The crude product was purified by chromatography (Cartridge Biotage Sfär 25 g 20 µm silica, Cyclohexane/EtOAc 100-0 / 4 CV, 100-0 to 80-20 / 12 CV, 80-20 / 8 CV, flow rate 80 ml / min) to give tert-butyl 3-(3-bromo-4-chloro-phenyl)-3-hydroxy-azetidine-1-carboxylate (1.25 g, 56.1%). ¹H NMR (500 MHz, DMSO-*d₆*) δ ppm 7.81 (d, *J*=2.20 Hz, 1 H), 7.64 (d, *J*=8.39 Hz, 1 H), 7.52 (dd, *J*=8.39, 2.20 Hz, 1 H), 6.57 (s, 1 H), 4.02 (s, 4 H), 1.41 (s, 9 H).

### Step d: tert-butyl 3-[4-chloro-3-[6-cyano-2-fluoro-3-(2-methoxyethoxy)phenyl]phenyl]-3-hydroxy-azetidine-1-carboxylate

In a MW vial, under argon, a solution of tert-butyl 3-(3-bromo-4-chloro-phenyl)-3-hydroxy-azetidine-1-carboxylate (300.0 mg, 0.78 mmol, 1 eq.) and 3-Fluoro-4-(2-methoxyethoxy)-2-(4,4,5,5-tetramethyl-1,3,2-dioxaborolan-2-yl)benzonitrile (841 mg, 2.36 mmol, 3 eq.) in toluene (9 mL) and water (1.65 mL) was degassed for 2 min. Then K₃PO₄ (667.3 mg, 3.144 mmol, 4 eq.), XPHOS PD G2 (61.9 mg, 0.0787 mmol, 0.100) and 2-dicyclohexylphosphino-2',6'-di-i-propoxy-1,1'-biphenyl (36.7 mg, 0.0786 mmol, 0.100) were added and the mixture was degassed for 5 min before being irradiated at 100°C for 2 h. The mixture was diluted with water and extracted with EtOAc (x3). The combined organic layers were dried over anhydrous MgSO₄, filtered and concentrated under reduced pressure. The crude product was purified by chromatography (Cartridge Biotage Sfär 25 g 20 µm silica, Cyclohexane/ E 100-0 / 4 CV, 100-0 to 50-50 / 20 CV, 50-50 / 6 CV, flow rate 80 ml / min) to give tert-butyl 3-[4-chloro-3-[6-cyano-2-fluoro-3-(2-methoxyethoxy)phenyl]phenyl]-3-hydroxy-azetidine-1-carboxylate (166 mg, 42.5%) as brown amorphous powder. ¹H NMR (400 MHz, DMSO-*d₆*) δ ppm 7.82 (dd, *J*=8.69, 1.43 Hz, 1 H), 7.68 (m, 2 H), 7.59 (dd, *J*=1.82, 0.83 Hz, 1 H), 7.46 (t, *J*=8.47 Hz, 1 H), 6.56 (s, 1 H), 4.35 (q, *J*=4.14 Hz, 2 H), 4.05 (m, 4 H), 3.72 (dd, *J*=4.95, 3.85 Hz, 2 H), 3.32 (s, 3 H), 1.39 (s, 9 H).

### Step e: 2-[2-chloro-5-(3-phenylazetidin-3-yl)phenyl]-3-fluoro-4-(2-methoxyethoxy)benzonitrile

In a 5 mL round bottom flask, AlCl₃ (129 mg, 0.97 mmol, 3 eq.) was suspended in benzene (3 mL) and cooled to 10°C under argon, tert-butyl 3-[4-chloro-3-[6-cyano-2-fluoro-3-(2-methoxyethoxy)phenyl]phenyl]-3-hydroxy-azetidine-1-carboxylate (160 mg, 0.322 mmol, 1 eq.) was dissolved in benzene (3 mL, 33.8 mmol, 105) and added to the reaction mixture. The reaction mixture was then stirred at 10°C for 1h. AlCl₃ (129 mg, 0.97 mmol, 3 eq.) was added again to the mixture which was stirred at rt for 90min. The reaction was quenched with cold water and stirred for 10 min. A saturated solution of NaHCO₃ was added and the mixture was extracted with EtOAc. The organic layer was dried over MgSO₄, filtered and the solvent removed under vacuum. The crude product was purified by chromatography (Cartridge Biotage Sfär 5 g 20 µm silica, DCM/MeOH 100-0 / 4 CV, 100-0 to 90-10 / 15 CV, 90-10 / 6 CV, flow rate 18 ml / min) to give 2-[2-chloro-5-(3-phenylazetidin-3-yl)phenyl]-3-fluoro-4-(2-methoxyethoxy)benzonitrile (93 mg, 66.1%) as a white powder. ¹H NMR (500 MHz, DMSO-*d₆*) δ ppm 7.81 (dd, *J*=8.67, 0.96 Hz, 1 H), 7.59 (d, *J*=8.39 Hz, 1 H), 7.53 (d, *J*=2.34 Hz, 1 H), 7.45 (d, *J*=8.53 Hz, 1 H), 7.42 (dd, *J*=8.39, 2.75 Hz, 1 H), 7.33 (m, 4 H), 7.20 (tt, *J*=6.64, 1.89 Hz, 1 H), 4.34 (m, 2 H), 4.14 (d, *J*=7.57 Hz, 1 H), 4.11 (d, *J*=7.70 Hz, 1 H), 4.08 (d, *J*=6.19 Hz, 1 H), 4.06 (d, *J*=6.19 Hz, 1 H), 3.71 (t, *J*=4.40 Hz, 2 H), 3.31 (s, 3 H).

### Step f: 2-[2-chloro-5-(3-phenylazetidin-3-yl)phenyl]-3-fluoro-4-(2-methoxyethoxy)benzamide

Into a 10 mL round bottom flask, under argon atmosphere, 2-[2-chloro-5-(3-phenylazetidin-3-yl)phenyl]-3-fluoro-4-(2-methoxyethoxy)benzonitrile (74 mg, 0.169 mmol, 1 eq.) was dissolved in EtOH (2.22 mL) and water (0.74 mL), then Ghaffar-Parkins catalyst (7.3 mg, 0.017 mmol, 0.10 eq.) was added. The reaction mixture was heated and stirred at 80°C for 2.5 h. The reaction mixture was concentrated under reduced pressure. The crude product was purified by chromatography (Cartridge Biotage Sfär 5 g 20 µm silica, DCM/MeOH (1%NH3) 100-0 / 4 CV, 100-0 to 90-10 / 15 CV, 90-10 / 6 CV, flow rate 18 ml / min) to give 2-[2-chloro-5-(3-phenylazetidin-3-yl)phenyl]-3-fluoro-4-(2-methoxyethoxy)benzamide (69.0 mg, 89.6%) as a white powder. ¹H NMR (500 MHz, DMSO-*d₆*) δ ppm 7.52 (br s, 1 H), 7.41 (d, *J*=8.39 Hz, 1 H), 7.37 (dd, *J*=8.53, 1.10 Hz, 1 H), 7.32 (m, 4 H), 7.28 (d, *J*=2.20 Hz, 1 H), 7.26 (d, *J*=8.39 Hz, 1 H), 7.23 (dd, *J*=8.39, 2.48 Hz, 1 H), 7.19 (tt, *J*=5.74, 2.79 Hz, 1 H), 7.12 (br s, 1 H), 4.24 (m, 2 H), 4.12 (d, *J*=7.43 Hz, 1 H), 4.11 (d, *J*=7.43 Hz, 1 H), 4.04 (d, *J*=7.50 Hz, 1 H), 4.03 (d, *J*=7.50 Hz, 1 H), 3.69 (t, *J*=4.54 Hz, 2 H), 3.31 (s, 3 H).

### Example 2: 2-[2-chloro-5-(3-phenylazetidin-3-yl)phenyl]-3-methyl-benzamide

### Step a: 3-methyl-2-(4,4,5,5-tetramethyl-1,3,2-dioxaborolan-2-yl)benzonitrile

The compound was prepared using the same procedure as detailed in example 1 step b starting from 2-bromo-3-methylbenzonitrile (2.50 g, 12.8 mmol, 1 eq.) giving 3-methyl-2-(4,4,5,5-tetramethyl-1,3,2-dioxaborolan-2-yl)benzonitrile (1.9 g, 45%) as a white powder. ¹H NMR (400 MHz, DMSO-*d₆*) δ ppm 7.63 (m, 1 H), 7.51 (m, 2 H), 3.31 (s, 6 H), 2.41 (s, 3 H).

### Step b: tert-butyl 3-[4-chloro-3-(2-cyano-6-methyl-phenyl)phenyl]-3-hydroxy-azetidine-1-carboxylate

The compound was prepared using the same procedure as detailed in example 1 step d starting from tert-butyl 3-(3-bromo-4-chloro-phenyl)-3-hydroxy-azetidine-1-carboxylate (Ex 1, step c) (320 mg, 0.86 mmol, 1 eq.) and 3-methyl-2-(4,4,5,5-tetramethyl-1,3,2-dioxaborolan-2-yl)benzonitrile (629 mg, 2.58 mmol, 3 eq.) giving tert-butyl 3-[4-chloro-3-(2-cyano-6-methylphenyl)phenyl]-3-hydroxy-azetidine-1-carboxylate (405 mg, quant) as a yellow powder. ¹H NMR (400 MHz, DMSO-*d₆*) δ ppm 7.79 (dd, *J*=7.70, 1.87 Hz, 1 H), 7.70 (dd, *J*=7.76, 1.93 Hz, 1 H), 7.67 (d, *J*=8.58 Hz, 1 H), 7.63 (dd, *J*=8.36, 2.31 Hz, 1 H), 7.54 (t, *J*=7.81 Hz, 1 H), 7.43 (d, *J*=1.76 Hz, 1 H), 6.54 (s, 1 H), 4.05 (m, 4 H), 2.07 (s, 3 H), 1.39 (s, 9 H).

### Step c: 2-[2-chloro-5-(3-phenylazetidin-3-yl)phenyl]-3-methyl-benzonitrile

The compound was prepared using the same procedure detailed in example 1 step e starting from tert-butyl 3-[4-chloro-3-(2-cyano-6-methyl-phenyl)phenyl]-3-hydroxy-azetidine-1-carboxylate (200 mg, 0.426 mmol, 1 eq.) giving 2-[2-chloro-5-(3-phenylazetidin-3-yl)phenyl]-3-methyl-benzonitrile (68.0 mg, 48.4%) as a yellow powder. ¹H NMR (400 MHz, DMSO-*d₆*) δ ppm 7.78 (dd, *J*=7.70, 1.87 Hz, 1 H), 7.68 (dd, *J*=7.76, 1.93 Hz, 1 H), 7.57 (dd, *J*=7.59, 0.88 Hz, 1 H), 7.52 (t, *J=*7.70 Hz, 1 H), 7.34 (m, 5 H), 7.20 (m, 2 H), 4.11 (d, *J*=7.37, 3.52 Hz, 1 H), 4.10 (d, *J*=7.37, 3.52 Hz, 1 H), 4.07 (d, *J*=7.92 Hz, 2 H), 2.02 (s, 3 H), 1.24 (br s, 1 H).

### Step d: 2-[2-chloro-5-(3-phenylazetidin-3-yl)phenyl]-3-methyl-benzamide

The compound was prepared using the same procedure as detailed in example 1 step f starting from 2-[2-chloro-5-(3-phenylazetidin-3-yl)phenyl]-3-methyl-benzonitrile (90.0 mg, 0.251 mmol, 1 eq.) giving 2-[2-chloro-5-(3-phenylazetidin-3-yl)phenyl]-3-methyl-benzamide (48.0 mg, 50.8%) as a white powder. ¹H NMR (500 MHz, DMSO-*d₆*) δ ppm 7.43 (br s, 1 H), 7.39 (d, *J*=8.67 Hz, 1 H), 7.33 (m, 8 H), 7.23 (br d, *J*=1.65 Hz, 1 H), 7.17 (br d, *J*=1.65 Hz, 3 H), 7.06 (br s, 1 H), 4.16 (d, *J*=7.70 Hz, 1 H), 4.14 (d, *J*=7.70 Hz, 1 H), 4.07 (br d, *J*=7.70 Hz, 1 H), 4.02 (br d, *J*=7.70 Hz, 1 H), 1.92 (s, 3 H).

### Example 3a and 3b: rel-(2aR)-2-[6-chloro-2-methyl-3-(3-phenylazetidin-3-yl)phenyl]-3-fluoro-4-(2-methoxyethoxy)benzamide and rel-(2aS)-2-[6-chloro-2-methyl-3-(3-phenylazetidin-3-yl)phenyl]-3-fluoro-4-(2-methoxyethoxy)benzamide

### Step a: 3-fluoro-4-(2-methoxyethoxy)-2-trimethylstannyl-benzonitrile

2-bromo-3-fluoro-4-(2-methoxyethoxy)benzonitrile (Ex 1, step a) (6 g, 21.89 mmol, 1 eq.) was dissolved in THF (60 mL). The solution was cooled down to -78°C then n-BuLi 1.6M in hexane (16 mL, 26 mmol, 1.2 eq.) was slowly added. The mixture became red wine. The reaction mixture was stirred at -78°C for 1h. Me₃SnCl (33 mL, 33 mmol, 1.5 eq.) was added as a solid. The reaction mixture was stirred at -78°C for 30 min. The reaction mixture was quenched at-78°C with a saturated solution of NH₄Cl. The reaction mixture was extracted with EtOAc (x3), dried over MgSO₄, filtered and concentrated to dryness. The crude product was purified by chromatography (Cartridge 100g silica kp, gradient = cyclohexane/EtOAc 9:1 (6cv) to 80:20 (9cv) and 80:20 (5cv), flow rate 40ml/min) to give 3-fluoro-4-(2-methoxyethoxy)-2-trimethylstannyl-benzonitrile (6.25 g, 79.8%) as a colorless oil. ¹H NMR (400 MHz, DMSO-*d₆*) δ ppm 7.63 (dd, *J*=8.36, 0.66 Hz, 1 H), 7.27 (t, *J*=8.80 Hz, 1 H), 4.24 (m, 2 H), 3.68 (m, 2 H), 3.31 (s, 3 H), 0.44 (m, 9 H).

### Step b: 3-Bromo-4-chloro-2-methylaniline

3-Bromo-2-methylaniline (10.00 g, 53.75 mmol, 1 eq.) was dissolved in dry DMF (54.0 mL) and NCS (7.54 g, 56.44 mmol, 1.05 eq.) was added in portions at rt. The resultant mixture was stirred at 50°C for 3 h. After completion, the mixture was diluted with water and extracted with EtOAc (3 times). The combined organic layers were washed with brine (three times), dried over anhydrous Na₂SO₄, and purified by column chromatography (eluent: 0-30% EtOAc in cyclohexane) to obtain 3-Bromo-4-chloro-2-methylaniline (5.50 g, 46%) as a brown solid. ¹H NMR (400 MHz, DMSO-*d₆*) δ 7.10 (dd, *J* = 8.7, 0.6 Hz, 1 H), 6.63 (d, *J* = 8.6 Hz, 1 H), 5.33 (s, 2 H), 2.21 (s, 3 H).

### Step c: 2-Bromo-1-chloro-4-iodo-3-methylbenzene

A solution was prepared from H₂O (17.4 mL) and H₂SO₄ (17.4 mL) while cooling in an ice bath and 3-Bromo-4-chloro-2-methylaniline (1.00 g, 4.54 mmol, 1 eq.), dissolved in ACN (17.4 mL) was added. The mixture was stirred for 10 min, then a solution of NaNO₂ (563 mg, 8.16 mmol, 1.8 eq.) in water (4.1 mL) was added dropwise, while keeping the temperature below 10°C. The mixture was stirred for 10 min then an ice-cold solution of KI (2.64 g, 15.87 mmol, 3.5 eq.) in water (7.9 mL) was added, and the resulting mixture was stirred at 0°C for 2 h. After completion, the mixture was diluted with CHCl₃, the layers were separated, and the aqueous layer was extracted with CHCl₃. The combined organic layers were washed with a saturated aqueous solution of NaHCO₃ (twice) and an aqueous solution of Na₂S₂O₃, then dried over MgSO₄, filtered, and concentrated in *vacuo* keeping the temperature below 35°C. The crude product was dissolved in CHCl₃, the insoluble was filtered off, washed with CHCl₃ and the filtrate was concentrated and dried in the same manner as above. The solid product was diluted with EtOH (the product did not dissolve completely) and water was added. The suspension was sonicated then the product was filtered, washed with water, and dried in *vacuo* (below 35°C) to obtain 2-Bromo-1-chloro-4-iodo-3-methylbenzene (1.33 g 88% yield) as a pale brown solid. ¹H NMR (400 MHz, CDCl₃) δ 7.71 (d, *J* = 8.4 Hz, 1H), 7.01 (d, *J* = 8.5 Hz, 1H), 2.74 (s, 3H). mp.: 57-58 °C.

### Step d: tert-butyl 3-(3-bromo-4-chloro-2-methyl-phenyl)-3-hydroxy-azetidine-1-carboxylate

To a 25 mL round bottom flask, flame dried, under argon, 2-Bromo-1-chloro-4-iodo-3-methylbenzene (1.16 g, 3.50 mmol, 2 eq.) was dissolved in toluene (9 mL) (dry, Acroseal) and the mixture was cooled to -95°C (Acetone, liquid N2). n-BuLi 1.6M in hexane (2.2 mL, 3.5 mmol, 2 eq.) was added dropwise and the mixture was stirred at -95°C for 1h. A solution of 1-tert-butyl 3-oxoazetidine-1-carboxylate (300 mg, 1.75 mmol, 1 eq.) in toluene (4.5 mL) was added dropwise, then the mixture was left to return to rt and was stirred at rt for 1h. The mixture was quenched with a saturated solution of NH₄Cl and extracted with EtOAc (x3). The combined organic layers were dried over anhydrous MgSO₄. After filtration, the filtrate was concentrated under reduced pressure. The crude product was purified by chromatography (Cartridge Biotage Sfär 25 g 20 µm silica, Cyclohexane/ E 100-0 / 4 CV, 100-0 to 80-20 / 15 CV, 80/20 / 8 CV, flow rate 80 ml / min) to give tert-butyl 3-(3-bromo-4-chloro-2-methylphenyl)-3-hydroxy-azetidine-1-carboxylate (258 mg, 27.4%) as a white powder.¹H NMR (400 MHz, DMSO-*d₆*) δ ppm 7.44 (dd, *J*=8.58, 0.55 Hz, 1 H), 7.37 (d, *J*=8.36 Hz, 1 H), 6.35 (s, 1 H), 4.34 (d, *J*=9.24 Hz, 2 H), 4.01 (br d, *J*=9.13 Hz, 2 H), 2.36 (s, 3 H), 1.38 (s, 9 H).

### Step e: tert-butyl 3-[4-chloro-3-[6-cyano-2-fluoro-3-(2-methoxyethoxy)phenyl]-2-methylphenyl]-3-hydroxy-azetidine-1-carboxylate

In a 100 mL round bottom flask, under argon, tert-butyl 3-(3-bromo-4-chloro-2-methylphenyl)-3-hydroxy-azetidine-1-carboxylate (467 mg, 0.868 mmol, 1 eq.), 3-fluoro-4-(2-methoxyethoxy)-2-trimethylstannyl-benzonitrile (621 mg, 1.73 mmol, 2 eq.), Bis(tri-t-butylphosphine)palladium(0) (222 mg, 0.434 mmol, 0.5 eq.) in DMF (31 mL) were degassed under argon and heated at 100°C for 90 min. The reaction mixture was diluted with water and extracted with EtOAc (3x). The organic layer was dried over MgSO₄, filtered and concentrated under reduced pressure. The crude was purified by chromatography (Cartridge Biotage Sfär 25 g 20 µm silica, Cyclohexane/E 100-0 / 4 CV, 100-0 to 60-40 / 15 CV, 60-40 / 8 CV, flow rate 80 ml / min) to give tert-butyl 3-[4-chloro-3-[6-cyano-2-fluoro-3-(2-methoxyethoxy)phenyl]-2-methyl-phenyl]-3-hydroxy-azetidine-1-carboxylate (160 mg, 33.8%) as a pale yellow powder. ¹H NMR (400 MHz, DMSO-*d₆*) δ ppm 7.86 (dd, *J*=8.64, 1.16 Hz, 1 H), 7.51 (d, , *J*=8.58 Hz, 1 H), 7.48 (d, , *J*=8.58 Hz, 1 H), 7.47 (t, , *J*=8.36 Hz, 1 H), 6.40 (s, 1 H), 4.40 (br d, *J*=9.46 Hz, 1 H), 4.35 (m, 2 H), 4.30 (d, *J*=9.35 Hz, 1 H), 4.03 (m, 2 H), 3.72 (m, 2 H), 3.32 and , 3.31 (s, 3 H), 1.99 and 1.98 (s, 3 H), 1.39 (s, 9 H).

### Step f: 2-[6-chloro-2-methyl-3-(3-phenylazetidin-3-yl)phenyl]-3-fluoro-4-(2-methoxyethoxy)benzonitrile

The compound was prepared using the same procedure as detailed in example 1 step e starting from tert-butyl 3-[4-chloro-3-[6-cyano-2-fluoro-3-(2-methoxyethoxy)phenyl]-2-methylphenyl]-3-hydroxy-azetidine-1-carboxylate (159 mg, 0.291 mmol, 1 eq.) giving 2-[6-chloro-2-methyl-3-(3-phenylazetidin-3-yl)phenyl]-3-fluoro-4-(2-methoxyethoxy)benzonitrile (94.0 mg, 71.5%) as a pale yellow powder. ¹H NMR (400 MHz, DMSO-*d₆*) δ ppm 7.83 (dd, *J*=8.64, 1.38 Hz, 1 H), 7.58 (d, *J*=8.36 Hz, 1 H), 7.42 (m, 4 H), 7.30 (m, 2 H), 7.22 (tt, *J*=7.37, 1.21 Hz, 1 H), 4.33 (m, 2 H), 4.31 (d, *J*=7.48 Hz, 2 H), 3.79 (d, *J*=6.82 Hz, 1 H), 3.75 (d, *J*=6.82 Hz, 1 H), 3.71 (dd, *J*=5.28, 3.52 Hz, 2 H), 3.30 (s, 3 H), 1.53 (s, 3 H).

### Step g: rel-(2aR)-2-[6-chloro-2-methyl-3-(3-phenylazetidin-3-yl)phenyl]-3-fluoro-4-(2-methoxyethoxy)benzamide and rel-(2aS)-2-[6-chloro-2-methyl-3-(3-phenylazetidin-3-yl)phenyl]-3-fluoro-4-(2-methoxyethoxy)benzamide

The compounds were prepared using the same procedure as detailed in example 1 step f starting from 2-[6-chloro-2-methyl-3-(3-phenylazetidin-3-yl)phenyl]-3-fluoro-4-(2-methoxyethoxy)benzonitrile (92.0 mg, 0.204 mmol, 1 eq) giving (56.0 mg, 58.5%). The two atropoisomers were separated by chiral chromatography (Column : Chiralpak IC, 250 *20mm*5µm, Mobile phase: 93% MtBE +0.1% Ethanolamine/ 7% MeOH +0.1% Ethanolamine, Flowrate : 35ml/min, T° column : 35°C , λ = 260nm) to give 3a rel-(2aR)-2-[6-chloro-2-methyl-3-(3-phenylazetidin-3-yl)phenyl]-3-fluoro-4-(2-methoxyethoxy)benzamide (7.0 mg, 18 %) as a pale-yellow powder. ¹H NMR (500 MHz, DMSO-*d₆*) δ ppm 7.47 (dd, *J*=8.39, 1.38 Hz, 1 H), 7.45 (d, J=7.84 Hz, 2 H), 7.35 (d, *J*=8.25 Hz, 1 H), 7.28 (m, 2 H), 7.22 (t, *J*=8.67 Hz, 1 H), 7.19 (d, *J*=8.53 Hz, 1 H), 7.17 (t, *J*=7.43 Hz, 1 H), 6.84 (m, 2 H), 4.31 (d, *J*=7.29 Hz, 1 H), 4.30 (d, *J*=7.29 Hz, 1 H), 4.24 (dd, *J*=5.16, 4.06 Hz, 2 H), 3.81 (d, *J*=7.29 Hz, 1 H), 3.76 (d, *J*=7.29 Hz, 1 H), 3.69 (m, 2 H), 3.31 (s, 3 H), 1.52 (s, 3 H); ee (method D) 98 %. And **3b** rel-(2aS)-2-[6-chloro-2-methyl-3-(3-phenylazetidin-3-yl)phenyl]-3-fluoro-4-(2-methoxyethoxy)benzamide (8.0 mg, 20 %) as a pale-yellow powder. ¹H NMR (500 MHz, DMSO-*d₆*) δ ppm 7.50 (br s, 1 H), 7.46 (m, 3 H), 7.37 (d, *J*=8.39 Hz, 1 H), 7.30 (d, *J*=7.70 Hz, 1 H), 7.27 (br d, *J*=5.36 Hz, 1 H), 7.24 (d, *J*=8.67 Hz, 1 H), 7.20 (t, *J*=7.43 Hz, 1 H), 7.16 (d, *J*=8.39 Hz, 1 H), 7.06 (br s, 1 H), 4.31 (d, *J*=7.29 Hz, 1 H), 4.28 (d, *J*=7.29 Hz, 1 H), 4.24 (dd, *J*=5.16, 3.09 Hz, 2 H), 3.75 (d, *J*=6.88 Hz, 1 H), 3.70 (m, 3 H), 3.30 (s, 3 H), 1.50 (s, 3 H); ee (method D) 98 %.

### Example 4a and 4b: (2aR)-2-[6-chloro-2-fluoro-3-(3-phenylazetidin-3-yl)phenyl]-3-fluoro-4-(2-methoxyethoxy)benzamide and (2aS)-2-[6-chloro-2-fluoro-3-(3-phenylazetidin-3-yl)phenyl]-3-fluoro-4-(2-methoxyethoxy)benzamide

### Step a: 2-Bromo-1-chloro-3-fluoro-4-iodobenzene

To a stirred solution of 2,2,6,6-tetramethylpiperidine (2.16 mL, 12.87 mmol, 2.2 eq.) in anhydrous THF (14.0 mL) was added n-BuLi (2.5 M solution in hexane, 5.15 mL, 12.87 mmol, 2.2 eq.) at -20°C under argon atmosphere. After 30 min of stirring at this temperature, the mixture was cooled to -78°C and 4-chloro-2-fluoro-1-iodobenzene (1.5 g, 5.85 mmol, 1 eq.) in anhydrous THF (5.85 mL) was added dropwise at a rate to maintain the temperature below -70°C, and the mixture was stirred for 2 h. Then, ZnCl₂ (1 M solution in Et₂O, 7.02 mL, 7.02 mmol, 1.2 eq.) was added dropwise and the mixture was stirred for additional 30 min. Then, an excess of Br₂ (899.0 µL, 17.55 mmol, 3.0 eq.) was added dropwise at a rate to keep the temperature below -50 °C and the mixture was stirred for a further 1 h. After completion, the mixture was allowed to warm to 25°C and quenched with H₂O and extracted twice with MtBE. The combined organic layers were washed with 1M aqueous solution of HCl, an aqueous Na₂S₂O₃ solution and water, dried over Na₂SO₄ and concentrated *in vacuo.* The residue was purified by flash column chromatography (eluent: n-heptane) to obtain 2-Bromo-1-chloro-3-fluoro-4-iodobenzene (1.52 g, 77.5%) as a green wax. ¹H NMR (400 MHz, CDCl₃): δ 7.63 (dd, *J=* 8.6, 6.4 Hz, 1H), 7.05 (dd, *J=* 8.6, 1.5 Hz, 1H).

### Step b: tert-butyl 3-(3-bromo-4-chloro-2-fluoro-phenyl)-3-hydroxy-azetidine-1-carboxylate

The compound was prepared using the same procedure as detailed in example 3a step d starting from 2-bromo-1-chloro-3-fluoro-4-iodo-benzene (1.18 g, 3.52 mmol, 2 eq.) and 1-tert-butyl 3-oxoazetidine-1-carboxylate (300 mg, 1.75 mmol, 1 eq.) giving tert-butyl 3-(3-bromo-4-chloro-2-fluoro-phenyl)-3-hydroxy-azetidine-1-carboxylate (379 mg, 52.3%) as a brown amorphous powder. ¹H NMR (400 MHz, DMSO-*d₆*) δ ppm 7.54 (dd, *J*=8.36, 7.59 Hz, 1 H), 7.49 (dd, *J*=8.58, 0.88 Hz, 1 H), 6.62 (s, 1 H), 4.27 (br d, *J*=9.46 Hz, 2 H), 3.97 (br d, *J*=9.46, 2 H), 1.39 (s, 9 H).

### Step c: tert-butyl 3-[4-chloro-3-[6-cyano-2-fluoro-3-(2-methoxyethoxy)phenyl]-2-fluorophenyl]-3-hydroxy-azetidine-1-carboxylate

The compound was prepared using the same procedure as detailed in example 3a step e starting from tert-butyl 3-(3-bromo-4-chloro-2-fluoro-phenyl)-3-hydroxy-azetidine-1-carboxylate (634 mg, 1.53 mmol, 1 eq) and 3-fluoro-4-(2-methoxyethoxy)-2-trimethylstannyl-benzonitrile (Ex 3a, step a) (1.1 g, 3.1 mmol, 2 eq.) giving tert-butyl 3-[4-chloro-3-[6-cyano-2-fluoro-3-(2-methoxyethoxy)phenyl]-2-fluoro-phenyl]-3-hydroxy-azetidine-1-carboxylate (375 mg, 47.0%) as a beige powder¹H NMR (400 MHz, DMSO-*d₆*) δ ppm 7.90 (dd, *J*=8.75, 1.38 Hz, 1 H), 7.73 (t, *J*=8.47 Hz, 1 H), 7.59 (dd, *J*=8.53, 0.94 Hz, 1 H), 7.53 (t, *J*=8.47 Hz, 1 H), 6.68 (s, 1 H), 4.37 (m, 2 H), 4.27 (d, *J*=9.20 Hz, 1 H), 4.23 (d, *J*=9.20 Hz, 1 H), 4.00 (d, *J*=9.13 Hz, 2 H), 3.72 (m, 2 H), 3.32 (s, 3 H), 1.37 (s, 9 H)

### Step d: 2-[6-chloro-2-fluoro-3-(3-phenylazetidin-3-yl)phenyl]-3-fluoro-4-(2-methoxyethoxy)benzonitrile

The compound was prepared using the same procedure as detailed in example 1 step e starting from tert-butyl 3-[4-chloro-3-[6-cyano-2-fluoro-3-(2-methoxyethoxy)phenyl]-2-fluorophenyl]-3-hydroxy-azetidine-1-carboxylate (371 mg, 0.712 mmol, 1 eq.) giving 2-[6-chloro-2-fluoro-3-(3-phenylazetidin-3-yl)phenyl]-3-fluoro-4-(2-methoxyethoxy)benzonitrile (233 mg, 71.9%) as a yellow powder. ¹H NMR (400 MHz, DMSO-*d₆*) δ ppm 7.86 (dd, *J*=8.75, 1.38 Hz, 1 H), 7.66 (t, *J*=8.25 Hz, 1 H), 7.59 (d, *J*=8.58 Hz, 1 H), 7.49 (t, *J*=8.53 Hz, 1 H), 7.43 (d, *J*=7.59 Hz, 2 H), 7.32 (m, 2 H), 7.21 (tt, *J*=7.26, 1.87 Hz, 1 H), 4.34 (td, *J*=4.43, 2.48 Hz, 2 H), 4.25 (d, *J*=7.37 Hz, 1 H), 4.22 (d, *J*=7.59 Hz, 1 H), 3.96 (d, *J*=7.48 Hz, 1 H), 3.89 (d, *J*=7.70 Hz, 1 H), 3.70 (m, 2 H).

### Step e: (2aR)-2-[6-chloro-2-fluoro-3-(3-phenylazetidin-3-yl)phenyl]-3-fluoro-4-(2-methoxyethoxy)benzamide and (2aS)-2-[6-chloro-2-fluoro-3-(3-phenylazetidin-3-yl)phenyl]-3-fluoro-4-(2-methoxyethoxy)benzamide

The compounds were prepared using the same procedure as detailed in example 1 step f starting from 2-[6-chloro-2-fluoro-3-(3-phenylazetidin-3-yl)phenyl]-3-fluoro-4-(2-methoxyethoxy)benzonitrile (230 mg, 0.506 mmol, 1 eq) giving the crude (160 mg, 66.9 %) as a white powder. The two atropoisomers were separated by chiral chromatography (Column : Chiralpak IC, 250 *20mm*5µm, Mobile phase : 93% MtBE +0.1% NH₃/ 7% MeOH +0.1% NH₃, Flowrate : 35ml/min, T° column : 35°C , λ = 260nm) to give **4a** rel-(2aR)-2-[6-chloro-2-fluoro-3-(3-phenylazetidin-3-yl)phenyl]-3-fluoro-4-(2-methoxyethoxy)benzamide (41.0 mg, 41.8 %) as a pale yellow powder. ¹H NMR (500 MHz, DMSO-*d₆*) δ ppm 7.71 (br s, 1 H), 7.51 (d, *J*=8.53 Hz, 1 H), 7.45 (d, *J*=7.70 Hz, 2 H), 7.36 (m, 2 H), 7.30 (m, 3 H), 7.20 (t, *J*=7.29 Hz, 1 H), 7.08 (br s, 1 H), 4.25 (m, 2 H), 4.23 (dd, *J*=8.12 Hz, 1 H), 4.21 (dd, *J*=8.12 Hz, 1 H), 3.89 (br d, *J*=7.57 Hz, 1 H), 3.80 (br d, *J*=7.29 Hz, 1 H), 3.69 (t, *J*=4.40 Hz, 2 H), 3.30 (s, 3 H). [α]_{d}^{22.7} = +21.8° (C=0.42, DCM); ee (method D) 98.4 %. And **4b** rel-(2aS)-2-[6-chloro-2-fluoro-3-(3-phenylazetidin-3-yl)phenyl]-3-fluoro-4-(2-methoxyethoxy)benzamide (35.0 mg, 35.7%) as a pale yellow powder. ¹H NMR (500 MHz, DMSO-*d₆*) δ ppm 7.71 (br s, 1 H), 7.51 (d, *J*=8.53 Hz, 1 H), 7.45 (d, *J*=7.70 Hz, 2 H), 7.36 (m, 2 H), 7.30 (m, 3 H), 7.20 (t, *J*=7.29 Hz, 1 H), 7.08 (br s, 1 H), 4.26 (m, 2 H), 4.23 (d, *J*=8.25 Hz, 1 H), 4.21 (d, *J*=8.25 Hz, 1 H), 3.89 (br d, *J*=7.57 Hz, 1 H), 3.80 (br d, *J*=7.43 Hz, 1 H), 3.69 (t, *J*=4.33 Hz, 2 H), 3.30 (s, 3H). [α]_{d}^{22.8} = -22.7° (C=0.41, DCM); ee (method D) 99.5 %.

### Example 5: 2-[2-chloro-5-(1-methyl-3-phenyl-azetidin-3-yl)phenyl]-3-fluoro-4-(2-methoxyethoxy)benzamide

### Step a: 3-(3-bromo-4-chloro-phenyl)-3-phenyl-azetidine

The compound was prepared using the same procedure as detailed in example 1 step e starting from tert-butyl 3-(3-bromo-4-chloro-phenyl)-3-hydroxy-azetidine-1-carboxylate (Ex 1, step c) (1.53 g, 4.22 mmol, 1.00 eq.) giving 3-(3-bromo-4-chloro-phenyl)-3-phenyl-azetidine (1.02 g, 74.9 %) as a yellow powder. ¹H NMR (400 MHz, DMSO-*d₆*) δ ppm 7.72 (d, *J*=2.20 Hz, 1 H), 7.55 (d, *J*=8.36 Hz, 1 H), 7.34 (m, 2 H), 7.30 (m, 3 H), 7.21 (m, 1 H), 4.10 (d, *J*=7.70 Hz, 2 H), 3.99 (d, *J*=7.70 Hz, 2 H).

### Step b: 3-(3-bromo-4-chloro-phenyl)-1-methyl-3-phenyl-azetidine

To a solution of 3-(3-bromo-4-chloro-phenyl)-3-phenyl-azetidine (500 mg, 1.55 mmol, 1.00 eq.) and DIPEA (768 µL, 4.65 mmol, 3.00 eq.) in ACN (20 mL), Mel (86 µL, 1.38 mmol, 0.891 eq.) was added. The mixture was stirred at 50 °C for 30min. The mixture was concentrated under reduced pressure. The crude was purified by chromatography (Cartridge Biotage Sfär 10 g 20 µm silica, DCM/MeOH (1%NH3) 100-0 / 4 CV, 100-0 to 98-2 / 15 CV, 98-2 / 6 CV, flow rate 40 mL / min) to give 3-(3-bromo-4-chloro-phenyl)-1-methyl-3-phenyl-azetidine (185 mg, 35.5 %) as a yellow gum. ¹H NMR (400 MHz, DMSO-*d₆*) δ ppm 7.74 (d, *J*=2.09 Hz, 1 H), 7.53 (d, *J*=8.36 Hz, 1 H), 7.36 (dd, *J*=8.36, 2.20 Hz, 1 H), 7.30 (m, 4 H), 7.20 (m, 1 H), 3.76 (m, 2 H), 3.67 (d, *J*=7.37 Hz, 2 H), 2.25 (s, 3 H).

### Step c: 2-[2-chloro-5-(1-methyl-3-phenyl-azetidin-3-yl)phenyl]-3-fluoro-4-(2-methoxyethoxy)benzonitrile

The compound was prepared using the same procedure as detailed in example 1 step d starting from 3-(3-bromo-4-chloro-phenyl)-1-methyl-3-phenyl-azetidine (50.0 mg, 0.149 mmol, 1.00 eq.) and 3-fluoro-4-(2-methoxyethoxy)-2-(4,4,5,5-tetramethyl-1,3,2-dioxaborolan-2-yl)benzonitrile (Ex 1, step b) (143 mg, 0.445 mmol, 3.00 eq.) giving 2-[2-chloro-5-(1-methyl-3-phenyl-azetidin-3-yl)phenyl]-3-fluoro-4-(2-methoxyethoxy)benzonitrile (66.0 mg, 88.7 %) as a yellow solid. ¹H NMR (400 MHz, DMSO-*d₆*) δ ppm 7.80 (m, 1 H), 7.57 (m, 1 H), 7.53 (d, *J*=1.87 Hz, 1 H), 7.45 (m, 2 H), 7.31 (m, 4 H), 7.19 (m, 1 H), 4.34 (m, 2 H), 3.92 and 3.91 (s, 3 H),3.90 (m, 2 H), 3.81 (m, 1 H), 3.77 (m, 2 H), 3.71 (m, 3 H), 2.28 and 2.26 (s, 3 H).

### Step d: 2-[2-chloro-5-(1-methyl-3-phenyl-azetidin-3-yl)phenyl]-3-fluoro-4-(2-methoxyethoxy)benzamide

The compound was prepared using the same procedure as detailed in example 1 step f starting from 2-[2-chloro-5-(1-methyl-3-phenyl-azetidin-3-yl)phenyl]-3-fluoro-4-(2-methoxyethoxy)benzonitrile (60.0 mg, 0.120 mmol, 1.00 eq.) giving 2-[2-chloro-5-(1-methyl-3-phenyl-azetidin-3-yl)phenyl]-3-fluoro-4-(2-methoxyethoxy)benzamide (18 mg, 31.9 %) as a white powder. ¹H NMR (400 MHz, DMSO-*d₆*) δ ppm 7.51 (br s, 1 H), 7.40 (d, *J*=8.58 Hz, 1 H), 7.37 (dd, *J*=8.69, 1.21 Hz, 1 H), 7.31 (d, *J*=0.55 Hz, 2 H), 7.30 (m, 2 H), 7.28 (m, 1 H), 7.25 (m, 2 H), 7.18 (m, 1 H), 7.11 (br s, 1 H), 4.24 (m, 2 H), 3.76 (m, 2 H), 3.69 (m, 4 H), 3.31 (s, 3 H), 2.25 (s, 3 H).

### Example 6a and 6b: rel-(2aR)-2-[2,3-dichloro-6-fluoro-5-(3-phenylazetidin-3-yl)phenyl]-3-fluoro-4-(2-methoxyethoxy)benzamide and rel-(2aS)-2-[2,3-dichloro-6-fluoro-5-(3-phenylazetidin-3-yl)phenyl]-3-fluoro-4-(2-methoxyethoxy)benzamide

### Step a: 3-bromo-4,5-dichloro-2-fluoroaniline

To a solution of 3-bromo-5-chloro-2-fluoroaniline (8.25 g, 36.8 mmol, 1.00 eq.) in DMF (36 mL) was added NCS (5.03 g, 37.7 mmol, 1.02 eq.) at room temperature. The reaction mixture was then stirred at 50°C for 5h. The reaction mixture was concentrated then diluted with water and extracted three times with EtOAc. The combined organic layers were washed with brine, dried over MgSO₄, filtered and concentrated to give a crude brown liquid which was purified by chromatography (Biotage Isolera Prime, Monitor 240 + 254 nm, Cartridge Biotage Sfar 50 g / 20 µm, Cyclohexane/EtOAc 100:0 over 5 CV, 100:0 to 90:10 over 30 CV, 90:10 over 5 CV, Flow rate 80 ml / min) to give an orange gum. This gum was triturated in pentane, and filtered to give 3-bromo-4,5-dichloro-2-fluoroaniline (2.88 g, 28.8 %) as a pink solid. ¹H NMR (400 MHz, DMSO-*d₆*) δ ppm 7.00 (d, *J*=8.36 Hz, 1 H), 5.89 (br s, 2 H).

### Step b: 3-bromo-1,2-dichloro-4-fluoro-5-iodo-benzene

To an ice-cold solution of water (28 mL), was added H₂SO₄ (28 mL). Then a solution of 3-bromo-4,5-dichloro-2-fluoroaniline (2 g, 7.34 mmol, 1.00 eq.) in ACN (28 mL) was added dropwise. The reaction mixture was stirred at 0°C for 10 min then a pre-cooled solution of NaNO₂ (911 mg, 13.2 mmol, 1.80 eq.) in water (7 mL) was added dropwise (while keeping the reaction temperature under 10°C). The mixture was stirred for 15 min then a pre-cooled solution of KI (4.26 g, 25.7 mmol, 3.50 eq.) in water (13 mL) was added dropwise. The mixture was then stirred at 0°C for 60min. The reaction mixture was diluted with CHCl₃, the aqueous phase was extracted three times with CHCl₃. The combined organic layers were washed with a saturated solution of NaHCO₃ (2x), then a solution of Na₂S₂O₃, dried over MgSO₄, filtered and concentrated to give a crude brown solid. The crude product was purified by chromatography (Cartridge Biotage Sfär 100 g 20 µm silica, Cyclohexane 100-0 / 10 CV, flow rate 30 ml / min) to give 3-bromo-1,2-dichloro-4-fluoro-5-iodo-benzene (2.25 g, 78.8%) as a white powder. LC/MS (method A) Rt = 2.60 min (95%). ¹H NMR (400 MHz, DMSO-*d₆*) δ ppm 8.24 (d, J=6.05 Hz, 1 H).

### Step c: tert-butyl 3-(3-bromo-4,5-dichloro-2-fluoro-phenyl)-3-hydroxy-azetidine-1-carboxylate

The compound was prepared using the same procedure as detailed in example 3a step d starting from 3-bromo-1,2-dichloro-4-fluoro-5-iodo-benzene (4 g, 10.8 mmol, 1.00 eq.) and 1-tert-butyl 3-oxoazetidine-1-carboxylate (3.2 g, 19 mmol, 1.7) giving tert-butyl 3-(3-bromo-4,5-dichloro-2-fluoro-phenyl)-3-hydroxy-azetidine-1-carboxylate (3.15 g, 67.2%) as a white powder. ¹H NMR (400 MHz, DMSO-*d₆*) δ ppm 7.78 (d, *J*=7.37 Hz, 1 H), 6.76 (br s, 1 H), 4.30 (d, *J*=8.91 Hz, 2 H), 3.95 (d, *J*=8.91 Hz, 2 H), 1.40 (s, 9 H).

### Step d: tert-butyl 3-[4,5-dichloro-3-[6-cyano-2-fluoro-3-(2-methoxyethoxy)phenyl]-2-fluorophenyl]-3-hydroxy-azetidine-1-carboxylate

The compound was prepared using the same procedure as detailed in example 3a step e starting from tert-butyl 3-(3-bromo-4,5-dichloro-2-fluoro-phenyl)-3-hydroxy-azetidine-1-carboxylate (2.00 g, 4.82 mmol, 1.00 eq.) and 3-fluoro-4-(2-methoxyethoxy)-2-trimethylstannyl-benzonitrile (Ex3a, step a) (2.69 g, 7.51 mmol, 1.56 eq.) giving tert-butyl 3-[4,5-dichloro-3-[6-cyano-2-fluoro-3-(2-methoxyethoxy)phenyl]-2-fluoro-phenyl]-3-hydroxy-azetidine-1-carboxylate (1.03 g, 38%) as a yellow powder. ¹H NMR (400 MHz, DMSO-*d₆*) δ ppm 7.98 (s, 1 H), 7.93 (d, *J*=8.80 Hz, 1 H), 7.56 (d, *J*=8.80 Hz, 1 H), 6.81 ( brs, 1 H), 4.38 (m, 2 H), 4.28 (m, 2 H), 3.99 (m, 2 H), 3.73 (m, 2 H), 3.32 (s, 3 H), 1.37 (s, 9 H).

### Step e: 2-[2,3-dichloro-6-fluoro-5-(3-phenylazetidin-3-yl)phenyl]-3-fluoro-4-(2-methoxyethoxy)benzonitrile

The compound was prepared using the same procedure as detailed in example 1 step e starting from tert-butyl 3-[4,5-dichloro-3-[6-cyano-2-fluoro-3-(2-methoxyethoxy)phenyl]-2-fluoro-phenyl]-3-hydroxy-azetidine-1-carboxylate (300 mg, 0.532 mmol, 1 eq.) giving 2-[2,3-dichloro-6-fluoro-5-(3-phenylazetidin-3-yl)phenyl]-3-fluoro-4-(2-methoxyethoxy)benzonitrile (140 mg, 53.7%) as pale-yellow powder. ¹H NMR (500 MHz, DMSO-*d₆*) δ ppm 8.05 (d, *J*=7.57 Hz, 1 H), 7.89 (d, *J*=8.67 Hz, 1 H), 7.53 (t, *J*=8.53 Hz, 1 H), 7.46 (d, *J*=7.84 Hz, 2 H), 7.33 (t, *J*=7.70 Hz, 2 H), 7.23 (t, *J*=6.74 Hz, 1 H), 4.35 (m, 2 H), 4.25 (d, *J*=7.70 Hz, 1 H), 4.20 (d, *J*=7.57 Hz, 1 H), 3.98 (d, *J*=7.70 Hz, 1 H), 3.90 (d, *J*=7.70 Hz, 1 H).

### Step f: rel-(2aR)-2-[2,3-dichloro-6-fluoro-5-(3-phenylazetidin-3-yl)phenyl]-3-fluoro-4-(2-methoxyethoxy)benzamide and rel-(2aS)-2-[2,3-dichloro-6-fluoro-5-(3-phenylazetidin-3-yl)phenyl]-3-fluoro-4-(2-methoxyethoxy)benzamide

The compound was prepared using the same procedure as detailed in example 1 step f starting from 2-[2,3-dichloro-6-fluoro-5-(3-phenylazetidin-3-yl)phenyl]-3-fluoro-4-(2-methoxyethoxy)benzonitrile (364 mg, 0.744 mmol, 1 eq.) giving a mixture of atropoisomers (186 mg, 48.7%) as a white powder. The two atropoisomers were separated by chiral chromatography (Column : Chiralpak IC, 250 *20mm*5µm, Mobile phase : 95% MtBE +0.1% NH₃/ 5% MeOH +0.1% nPropylamine, Flowrate : 35mL/min, T° column : 10°C , λ = 260nm) to give **6a** rel-(2aR)-2-[2,3-dichloro-6-fluoro-5-(3-phenylazetidin-3-yl)phenyl]-3-fluoro-4-(2-methoxyethoxy)benzamide (58 mg) as a white powder. ¹H NMR (500 MHz, DMSO-*d₆* + deuterated HCl) δ ppm 8.06 (d, *J*=7.56 Hz, 1 H), 7.61 (d, *J*=8.53 Hz, 1 H), 7.51 (d, *J*=7.84 Hz, 2 H), 7.41 (t, *J*=7.77 Hz, 2 H), 7.36 (t, *J*=8.60 Hz, 1 H), 7.32 (m, 1 H), 4.64 (br d, *J*= 11.42 Hz, 1 H), 4.51 (d, *J*=11.28 Hz, 1 H), 4.27 (m, 2 H), 3.69 (t, *J*=4.40 Hz, 2 H), 3.30 (s, 3 H). [α]_{d}^{21.3} = +9.7° (C=0.38, DCM) ee (method D) 99.5% (Rt = 10.3 min); and 6b rel-(2aS)-2-[2,3-dichloro-6-fluoro-5-(3-phenylazetidin-3-yl)phenyl]-3-fluoro-4-(2-methoxyethoxy)benzamide (44 mg) as a white powder. ¹H NMR (500 MHz, DMSO-*d₆*) δ ppm 7.74 (br s, 1 H), 7.69 (d, *J*=7.29 Hz, 1 H), 7.56 (dd, *J*=8.60, 1.03 Hz, 1 H), 7.46 (d, J=7.70 Hz, 2 H), 7.32 (m, 3 H), 7.21 (m, 1 H), 7.06 (br s, 1 H), 4.26 (m, 2 H), 4.22 (d, *J*=7.57 Hz, 1 H), 4.20 (d, *J*=7.84 Hz, 1 H), 3.92 (d, *J*=7.57 Hz, 1 H), 3.83 (d, *J*=7.70 Hz, 1 H), 3.69 (m, 2 H), 3.30 (m, 3 H). [α]_{d}^{21.3} = - 11.5° (C=0.41, DCM) ee (method D) 99.9% (Rt = 14.2 min).

### Example 7a and 7b: rel-(2aR)-2-[2,6-difluoro-3-(3-phenylazetidin-3-yl)phenyl]-3-fluoro-4-(2-methoxyethoxy) benzamide and rel-(2aS)-2-[2,6-difluoro-3-(3-phenylazetidin-3-yl)phenyl]-3-fluoro-4-(2-methoxyethoxy) benzamide

### Step a: tert-butyl 3-(3-bromo-2,4-difluoro-phenyl)-3-hydroxy-azetidine-1-carboxylate

The compound was prepared using the same procedure detailed in example 3a step d starting from 1-bromo-2,6-difluoro-3-iodobenzene (1.5 g, 4.7 mmol, 1.0 eq.) and 1- tert-butyl 3-oxoazetidine-1-carboxylate (1.2 g, 7.0 mmol, 1.5 eq.) giving tert-butyl 3-(3-bromo-2,4-difluoro-phenyl)-3-hydroxy-azetidine-1-carboxylate (1.31 g, 71 %) as a white powder. ¹H NMR (400 MHz, DMSO-*d₆*) δ ppm 7.57 (td, *J*=8.78, 6.33 Hz, 1 H), 7.26 (m, 1 H), 6.58 (s, 1 H), 4.27 (br d, *J*=9.02 Hz, 2 H), 3.97 (br d, *J*=9.13 Hz, 2 H), 1.39 (s, 9 H).

### Step b: tert-butyl 3-[3-[6-cyano-2-fluoro-3-(2-methoxyethoxy)phenyl]-2,4-difluoro-phenyl]-3-hydroxy-azetidine-1-carboxylate

The compound was prepared using the same procedure detailed in example 3a step e starting from tert-butyl 3-(3-bromo-2,4-difluoro-phenyl)-3-hydroxy-azetidine-1-carboxylate (1.1 g, 3.0 mmol, 1.0 eq.) and 3-fluoro-4-(2-methoxyethoxy)-2-trimethylstannyl-benzonitrile (Ex 3a, step a) (1.6 g, 4.5 mmol, 1.5 eq.) giving tert-butyl 3-[3-[6-cyano-2-fluoro-3-(2-methoxyethoxy)phenyl]-2,4-difluoro-phenyl]-3-hydroxy-azetidine-1-carboxylate (1.2 g, 73 %) as a yellow oil. ¹H NMR (400 MHz, DMSO-*d₆*) δ ppm 7.90 (dd, *J*=8.69, 1.43 Hz, 1 H), 7.77 (td, *J*=8.80, 6.93 Hz, 1 H), 7.53 (t, *J*=8.53 Hz, 1 H), 7.36 (t, *J*=8.31 Hz, 1 H), 6.63 (s, 1 H), 4.37 (m, 2 H), 4.30 (br d, *J*=9.02 Hz, 1 H), 4.26 (br d, *J*=9.02 Hz, 1 H), 4.01 (m, 2 H), 3.72 (m, 2 H), 3.32 (s, 3 H), 1.38 (s, 9 H).

### Step c: 2-[2,6-difluoro-3-(3-phenylazetidin-3-yl)phenyl]-3-fluoro-4-(2-methoxyethoxy) benzonitrile

The compound was prepared using the same procedure detailed in example 1 step e starting from tert-butyl 3-[3-[6-cyano-2-fluoro-3-(2-methoxyethoxy)phenyl]-2,4-difluoro-phenyl]-3-hydroxy-azetidine-1-carboxylate (1.2 g, 2.5 mmol, 1.0 eq.) giving 2-[2,6-difluoro-3-(3-phenylazetidin-3-yl)phenyl]-3-fluoro-4-(2-methoxyethoxy) benzonitrile (569 mg, 52 %) as a beige powder. ¹H NMR (400 MHz, DMSO-*d₆*) δ ppm 7.86 (dd, *J*=8.75, 1.49 Hz, 1 H), 7.70 (td, *J*=8.83, 6.44 Hz, 1 H), 7.50 (t, *J*=8.53 Hz, 1 H), 7.44 (d, *J*=7.48 Hz, 2 H), 7.38 (m, 1 H), 7.32 (m, 2 H), 7.21 (s, 1 H), 4.34 (m, 2 H), 4.28 (d, *J*=7.59 Hz, 1 H), 4.24 (d, *J*=7.48 Hz, 1 H), 4.01 (d, *J*=7.70 Hz, 1 H), 3.92 (d, *J*=7.48 Hz, 1 H), 3.70 (m, 2 H), 3.30 (s, 3 H).

### Step d: rel-(2aR)-2-[2,6-difluoro-3-(3-phenylazetidin-3-yl)phenyl]-3-fluoro-4-(2-methoxyethoxy)benzamide and rel-(2aS)-2-[2,6-difluoro-3-(3-phenylazetidin-3-yl)phenyl]-3-fluoro-4-(2-methoxyethoxy)benzamide

The compound was prepared using the same procedure detailed in example 1 step f starting from 2-[2,6-difluoro-3-(3-phenylazetidin-3-yl)phenyl]-3-fluoro-4-(2-methoxyethoxy) benzonitrile (569 mg, 1.30 mmol, 1.00 eq.) giving mixture of two atropoisomers (320 mg, 0.694 mmol, 0.535 eq., 53.5 %) as a white powder. The two atropoisomers were separated by chirale chromatography (Column : Chiralpak IC, 250 *20mm*5µm, Mobile phase : 95% MtBE +0.1% NH3/ 5% MeOH +0.1% nPropylamine, Flowrate : 35ml/min, T° column : 10°C , λ = 260nm) to give **7a** rel-(2aR)-2-[2,6-difluoro-3-(3-phenylazetidin-3-yl)phenyl]-3-fluoro-4-(2-methoxyethoxy) benzamide (87 mg, 14 %) as a white powder ¹H NMR (500 MHz, DMSO-*d₆*) δ ppm 7.73 (br s, 1 H), 7.48 (dd, J=8.60, 0.89 Hz, 1 H), 7.45 (d, *J*=7.57 Hz, 2 H), 7.40 (td, *J*=8.70, 6.53 Hz, 1 H), 7.31 (m, 3 H), 7.20 (m, 1 H), 7.13 (t, *J*=8.67 Hz, 1 H), 7.12 (br s, 1 H), 4.25 (m, 2 H), 4.24 (d, *J*=7.43 Hz, 1 H), 4.21 (d, *J*=7.57 Hz, 1 H), 3.91 (d, *J*=7.57 Hz, 1 H), 3.82 (d, *J*=7.43 Hz, 1 H), 3.69 (m, 2 H), 3.30 (s, 3 H). [α]_{d}^{23.5} = +19.3° (C=0.41, DCM) ee (method D) 98.8% (Rt = 9.9 min); and **7b** rel-(2aS)-2-[2,6-difluoro-3-(3-phenylazetidin-3-yl)phenyl]-3-fluoro-4-(2-methoxyethoxy) benzamide (71 mg, 11.8 %) as a white powder ¹H NMR (500 MHz, DMSO-*d₆*) δ ppm 7.73 (br s, 1 H), 7.48 (dd, *J*=8.60, 0.89 Hz, 1 H), 7.45 (d, *J*=7.57 Hz, 2 H), 7.40 (td, *J*=8.63, 6.53 Hz, 1 H), 7.31 (m, 3 H), 7.20 (m, 1 H), 7.13 (t, *J*=8.67 Hz, 1 H), 7.12 (br s, 1 H), 4.25 (m, 2 H), 4.24 (d, *J*=7.43 Hz, 1 H), 4.21 (d, *J*=7.57 Hz, 1 H), 3.91 (d, *J*=7.43 Hz, 1 H), 3.82 (d, *J*=7.43 Hz, 1 H), 3.69 (m, 2 H), 3.30 (s, 3 H). [α]_{d}^{23.7} = - 13.8° (C=0.36, DCM) ee (method D) 99.3% (Rt = 12.6 min).

### Example 8a and 8b: rel-(2aR)-2-[2,6-dichloro-3-(3-phenylazetidin-3-yl)phenyl]-3-fluoro-4-(2-methoxyethoxy)benzamide and rel-(2aS)-2-[2,6-dichloro-3-(3-phenylazetidin-3-yl)phenyl]-3-fluoro-4-(2-methoxyethoxy)benzamide

### Step a : 3-bromo-2,4-dichloro-aniline

To a solution of 3-bromo-2-chloro-phenylamine (10.0 g, 48.4 mmol, 1.0 eq.) in DMF (50 mL) was added NCS (7.44 g, 55.7 mmol, 1.15 eq.) at room temperature. The reaction mixture was then stirred at 50°C for 2.5H. The reaction mixture was concentrated then diluted with water and extracted three times with ethyl acetate. The combined organic layers were washed with brine, dried over MgSO4, filtered and concentrated. The crude product was purified by chromatography (Cartridge Biotage Sfär 200 g 60 µm silica, Cyclohexane/DCM, flow rate 200 ml / min to give 3-bromo-2,4-dichloro-aniline (7.28 g, 62.4 %) as a white powder. ¹H NMR (400 MHz, DMSO-*d₆*) δ ppm 7.25 (d, *J*=8.80 Hz, 1 H), 6.81 (d, *J*=8.80 Hz, 1 H), 5.82 (br s, 2 H).

### Step b : 2-bromo-1,3-dichloro-4-iodo-benzene

To an ice-cold solution of water (30 mL), was added H2SO4 (30 mL). Then a solution of 3-bromo-2,4-dichloro-aniline (2.00 g, 8.30 mmol, 1.0 eq.) in acetonitrile (30 mL) was added dropwise. The reaction mixture was stirred at 0°C for 10 min, then a pre-cooled solution of NaNO2 (1.03 g, 14.9 mmol, 1.8 eq.) in water (7.5 mL) was added dropwise (reaction must not exceed 10°C). The mixture was stirred for 15 min then a pre-cooled solution of KI (4.82 g, 29.0 mmol, 3.5 eq.) in water (14 mL) was added dropwise. The mixture was then stirred at 0°C for 90 min. The reaction mixture was diluted with CHCl3, the layers were separated and the aqueous layer was extracted three times with CHCl3. The combined organic layers were washed with a saturated solution of NaHCO3 (2x), then a solution of Na2S2O3(1x), dried over MgSO4, filtered and concentrated to give crude (yellow solid). The crude product was purified by chromatography (Cartridge Biotage Sfär 100 g 20 µm silicaCyclohexane 100-0/10 CV (column volume), flow rate 30 ml / min) to give 2-bromo-1,3-dichloro-4-iodo-benzene (2.20 g, 75.3 %). ¹H NMR (400 MHz, DMSO-*d₆*) δ ppm 7.98 (d, J=8.58 Hz, 1 H), 7.37 (d, J=8.47 Hz, 1 H).

### Step c : tert-butyl 3-(3-bromo-2,4-dichloro-phenyl)-3-hydroxy-azetidine-1-carboxylate

The compound was prepared using the same procedure detailed in example 1 step c starting from 1- tert-butyl 3-oxoazetidine-1-carboxylate (3.9 g, 23 mmol, 2.0 eq.) and 2-bromo-1,3-dichloro-4-iodo-benzene (4.00 g, 11.4 mmol, 1.00 eq.) giving tert-butyl 3-(3-bromo-2,4-dichloro-phenyl)-3-hydroxy-azetidine-1-carboxylate (2.63 g, 58.2 %) as a white powder. ¹H NMR (400 MHz, DMSO-*d₆*) δ ppm 7.61 (d, *J*=8.47 Hz, 1 H), 7.53 (d, *J*=8.47 Hz, 1 H), 6.43 (br s, 1 H), 4.38 (d, *J*=9.35 Hz, 2 H), 4.00 (m, 2 H), 1.38 (s, 9 H).

### Step d : tert-butyl 3-[2,4-dichloro-3-[6-cyano-2-fluoro-3-(2-methoxyethoxy)phenyl]phenyl]-3-hydroxy-azetidine-1-carboxylate

The compound was prepared using the same procedure detailed in example 3a step e starting from tert-butyl 3-(3-bromo-2,4-dichloro-phenyl)-3-hydroxy-azetidine-1-carboxylate (2.0 g, 5.04 mmol, 1.0 eq.) and 3-fluoro-4-(2-methoxyethoxy)-2-trimethylstannyl-benzonitrile (2.7 g, 7.5 mmol, 1.5 eq.) giving tert-butyl 3-[2,4-dichloro-3-[6-cyano-2-fluoro-3-(2-methoxyethoxy)phenyl]phenyl]-3-hydroxy-azetidine-1-carboxylate (437 mg, 15.8 %) as a pale yellow gum. ¹H NMR (400 MHz, DMSO-*d₆*) δ ppm 7.90 (dd, *J*=8.69, 1.43 Hz, 1 H), 7.71 (s, 2 H), 7.51 (s, 1 H), 6.52 ( br s, 1 H), 4.44 (d, *J*=9.46 Hz, 1 H), 4.37 (m, 3 H), 4.03 (m, 2 H), 3.73 (m, 2 H), 3.32 (s, 3 H), 1.38 (s, 9 H).

### Step e : 2-[2,6-dichloro-3-(3-phenylazetidin-3-yl)phenyl]-3-fluoro-4-(2-methoxyethoxy)benzonitrile

The compound was prepared using the same procedure detailed in example 1 step e starting from tert-butyl 3-[2,4-dichloro-3-[6-cyano-2-fluoro-3-(2-methoxyethoxy)phenyl]phenyl]-3-hydroxy-azetidine-1-carboxylate (230 mg, 0.450 mmol, 1.0 eq.) giving 2-[2,6-dichloro-3-(3-phenylazetidin-3-yl)phenyl]-3-fluoro-4-(2-methoxyethoxy)benzonitrile (133 mg, 61.5 %) as a pale-yellow solid. ¹H NMR (400 MHz, DMSO-*d₆*) δ ppm 7.86 (dd, *J*=8.64, 1.49 Hz, 1 H), 7.77 (d, *J*=8.47 Hz, 1 H), 7.65 (d, *J*=8.58 Hz, 1 H), 7.48 (m, 1 H), 7.44 (m, 2 H), 7.30 (m, 2 H), 7.22 (m, 1 H), 4.33 (m, 4 H), 3.86 (m, 1 H), 3.80 (m, 1 H), 3.70 (m, 2 H), 3.30 (s, 3 H).

### Step f : rel-(2aR)-2-[2,6-dichloro-3-(3-phenylazetidin-3-yl)phenyl]-3-fluoro-4-(2-methoxyethoxy)benzamide and rel-(2aS)-2-[2,6-dichloro-3-(3-phenylazetidin-3-yl)phenyl]-3-fluoro-4-(2-methoxyethoxy)benzamide

The compound was prepared using the same procedure detailed in example 1 step f starting from 2-[2,6-dichloro-3-(3-phenylazetidin-3-yl)phenyl]-3-fluoro-4-(2-methoxyethoxy)benzonitrile (130 mg, 0.276 mmol, 1.0 eq.) giving mixture of two atropoisomers (77.0 mg, 57.1 %). The two atropoisomers were separated by chirale chromatography (Column : Chiralpak IC, 250 *20mm*5µm, Mobile phase : 95% MtBE +0.1% NH3/ 5% MeOH +0.1% nPropylamine, Flowrate : 35ml/min, T° column : 10°C , λ = 260nm) to give **8a** rel-(2aR)-2-[2,6-dichloro-3-(3-phenylazetidin-3-yl)phenyl]-3-fluoro-4-(2-methoxyethoxy)benzamide (24 mg, 38 %) as white powder. ¹H NMR (500 MHz, DMSO-*d₆*) δ ppm 7.65 (br d, *J*=0.83 Hz, 1 H), 7.56 (m, 1 H), 7.53 (d, *J*=8.53 Hz, 1 H), 7.46 (dd, *J*=8.32, 1.17 Hz, 2 H), 7.37 (d, *J*=8.39 Hz, 1 H), 7.29 (m, 3 H), 7.19 (m, 1 H), 7.04 (br s, 1 H), 4.30 (d, *J*=7.57 Hz, 2 H), 4.25 (dd, *J*=5.78, 3.16 Hz, 2 H), 3.81 (d, *J*=7.15 Hz, 1 H), 3.75 (d, *J*=7.43 Hz, 1 H), 3.69 (m, 2 H), 3.31 (s, 3 H). [α]_{d}^{22.6} = +22.6° (C=0.34, DCM) ee (method D) 99.6% (Rt = 10.2 min) and **8b** rel-(2aS)-2-[2,6-dichloro-3-(3-phenylazetidin-3-yl)phenyl]-3-fluoro-4-(2-methoxyethoxy)benzamide (23 mg, 36.5 %) as a white powder. ¹H NMR (500 MHz, DMSO-*d₆*) δ ppm 7.65 (br s, 1 H), 7.56 (m, 1 H), 7.53 (d, *J*=8.53 Hz,, 1 H), 7.46 (dd, *J*=8.39, 1.10 Hz, 2 H), 7.37 (d, *J*=8.39 Hz, 1 H), 7.29 (m, 3 H), 7.19 (m, 1 H), 7.04 (br s, 1 H), 4.30 (d, *J*=7.70 Hz, 2 H), 4.25 (dd, *J*=5.57, 2.96 Hz, 2 H), 3.81 (d, *J*=7.29 Hz, 1 H), 3.75 (d, *J*=7.29 Hz, 1 H), 3.69 (m, 2 H), 3.31 (s, 3 H) [α]_{d}^{22.8} = -20.2° (C=0.35, DCM) ee (method D) 99.8% (Rt = 14.7 min)

### Example 9a and 9b: rel-(2aR)-3-chloro-2-[2-chloro-5-(3-phenylazetidin-3-yl)phenyl]-4-(2-methoxyethoxy)benzamide and rel-(2aS)-3-chloro-2-[2-chloro-5-(3-phenylazetidin-3-yl)phenyl]-4-(2-methoxyethoxy)benzamide

### Step a : tert-butyl 3-[4-chloro-3-(4,4,5,5-tetramethyl-1,3,2-dioxaborolan-2-yl)phenyl]-3-hydroxy-azetidine-1-carboxylate

In a 25mL round bottom Flask, to a solution of tert-butyl 3-(3-bromo-4-chloro-phenyl)-3-hydroxy-azetidine-1-carboxylate (Ex 1, step c) (2.35 g, 6.48 mmol, 1.0 eq.) and bis(pinacolato)diboron (4.94 g, 19.5 mmol, 3.0 eq.) in 1,4-dioxane (36 mL) was added AcOK (1.91 g, 19.5 mmol, 3.0 eq.) and Pd(dppf)Cl2 (474 mg, 0.6478 mmol, 0.1 eq.) under a nitrogen atmosphere. The mixture was stirred at 100 °C for 6h. The mixture was concentrated filtered on Whatman and washed with DCM and EtOAc. The filtrate was concentrated under reduced pressure to give a black gum. The crude was purified by chromatography (Cartridge Biotage Sfär 100 g 20 µm silica (Absorbance : 230nm), Cyclohexane/AcOEt 100-0 / 4 CV (column volume), 100-0 to 80-20 / 20 CV,80-20 / 10 CV, flow rate 80 ml / min) to give DTIR0910-002 (2.41 g, 72.6 %) as a pale-yellow powder. ¹H NMR (400 MHz, DMSO-*d₆*) δ ppm 7.75 (d, *J*=2.31 Hz, 1 H), 7.57 (dd, *J*=8.36, 2.53 Hz, 1 H), 7.43 (d, *J*=8.36 Hz, 1 H), 6.46 (s, 1 H), 4.01 (m, 4 H), 1.42 (s, 9 H), 1.40 (s, 3 H), 1.16 (s, 6 H), 1.07 (s, 3H).

### Step b : 3-chloro-4-(2-methoxyethoxy)benzonitrile

In a 100mL round bottom flask, was added 2-chloro-4-cyanophenol (5.00 g, 32.6 mmol, 1.0 eq.), K2CO3 (9 g, 65.1204 mmol, 2.00 eq.), 2-bromoethyl methyl ether (3.41 mL, 35.8 mmol, 1.1 eq.) and DMF (30 mL). The reaction mixture was stirred at 60°C overnight. The mixture was diluted with water and extracted with EtOAc (3 times). The combined organics layers were washed with a saturated solution of NaCl (x3), dried over MgSO4, filtered and concentrated to give 3-chloro-4-(2-methoxyethoxy)benzonitrile (6.56 g, 95.2 %) as a white powder. ¹H NMR (400 MHz, DMSO-*d₆*) δ ppm 8.02 (d, *J*=1.98 Hz, 1 H), 7.81 (dd, *J*=8.69, 2.09 Hz, 1 H), 7.34 (d, *J*=8.69 Hz, 1 H), 4.30 (m, 2 H), 3.71 (m, 2 H), 3.31 (s, 3 H).

### Step c : 3-chloro-2-iodo-4-(2-methoxyethoxy)benzonitrile

Under argon atmosphere, LDA (16.3 mL, 32.6 mmol, 2.30 eq.) was added to a solution of 3-chloro-4-(2-methoxyethoxy)benzonitrile (3.00 g, 14.2 mmol, 1.0 eq.) in dry tetrahydrofuran (36 mL) at -78 °C and stirred at this temperature for 45 min. Then, a solution of I2 (5.4 g, 21 mmol, 1.5 eq.) in dry tetrahydrofuran (6 mL) was added and stirring was continued at -78 °C for 1h. The mixture was quenched with a sat. aq. solution of NH₄Cl extracted with EtOAc (x2) and washed with H2O (x1) . The organic layer was dried over anhydrous MgSO4. After filtration, the filtrate was concentrated under reduced pressure to give crude as a brown solid. The crude was purified by chromatography (Cartridge Biotage Sfär 100 g 60 µm silica, Cyclohexane/DCM 90-10 / 4 CV (column volume), 100-0 to 60-40 / 20 CV, 60-40 / 10 CV, flow rate 100 ml / min) The product was washed with n-heptane (x2), n-pentane(x2) and diethyl ether (x1) to give 3-chloro-2-iodo-4-(2-methoxyethoxy)benzonitrile (3.66 g, 73.4 %) as a brown powder. ¹H NMR (400 MHz, DMSO-*d₆*) δ ppm 7.82 (d, *J*=8.69 Hz, 1 H), 7.35 (d, *J*=8.80 Hz, 1 H), 4.31 (m, 2 H), 3.71 (m, 2 H), 3.32 (s, 3 H).

### Step d : tert-butyl 3-[4-chloro-3-[2-chloro-6-cyano-3-(2-methoxyethoxy)phenyl]phenyl]-3-hydroxy-azetidine-1-carboxylate

In a 100mL round bottom flask, under argon, a solution of tert-butyl 3-[4-chloro-3-(4,4,5,5-tetramethyl-1,3,2-dioxaborolan-2-yl)phenyl]-3-hydroxy-azetidine-1-carboxylate (1.70 g, 3.32 mmol, 1.0 eq.) and 3-chloro-2-iodo-4-(2-methoxyethoxy)benzonitrile (1.34 g, 3.97 mmol, 1.2 eq.) in toluene (34 mL, 320 mmol, 96.4)/WATER (6.8 mL, 380 mmol, 110) was degassed for 2 min. Then Pd2(dba)3 (152 mg, 0.166 mmol, 0.05 eq.), N-XANTPHOS (183 mg, 0.3318 mmol, 0.1 eq.) and K3PO4 (2.11 g, 9.94 mmol, 2.99 eq.) were added and the mixture was degassed for 2 min before being stirred at 100°C for 8h. The mixture was diluted with saturated solution of NaHCO3 and extracted with EtOAc (x3). The combined organic layers were dried over anhydrous MgSO4. After filtration, the filtrate was concentrated under reduced pressure to give 3 g of crude as a green gum. The crude product was purified by chromatography (Cartridge Biotage Sfär 100 g 20 µm silica, Cyclohexane/AcOEt 100-0 / 4 CV (column volume) 100-0 to 65-35 / 17 CV 65-35 / 10 CV, flow rate 90 ml / min) to give tert-butyl 3-[4-chloro-3-[2-chloro-6-cyano-3-(2-methoxyethoxy)phenyl]phenyl]-3-hydroxy-azetidine-1-carboxylate (684 mg, 36.3 %) as a yellow powder.¹H NMR (400 MHz, DMSO-*d₆*) δ ppm 7.96 (d, *J*=8.80 Hz, 1 H), 7.66 (m, 2 H), 7.49 (dd, *J*=1.87, 0.77 Hz, 1 H), 7.42 (d, *J*=8.80 Hz, 1 H), 6.56 (s, 1 H), 4.36 (m, 2 H), 4.04 (m, 4 H), 3.74 (m, 2 H), 3.34 (s, 3 H), 1.39 (s, 9 H).

### Step e : 3-chloro-2-[2-chloro-5-(3-phenylazetidin-3-yl)phenyl]-4-(2-methoxyethoxy)benzonitrile

The compound was prepared using the same procedure detailed in example 1 step e starting from tert-butyl 3-[4-chloro-3-[2-chloro-6-cyano-3-(2-methoxyethoxy)phenyl]phenyl]-3-hydroxy-azetidine-1-carboxylate (374 mg, 0.758 mmol, 1.0 eq.) giving 3-chloro-2-[2-chloro-5-(3-phenylazetidin-3-yl)phenyl]-4-(2-methoxyethoxy)benzonitrile (210 mg, 61 %) as pale-yellow powder. ¹H NMR (400 MHz, DMSO-*d₆*) δ ppm 7.94 (d, *J*=8.69 Hz, 1 H), 7.57 (d, *J*=8.36 Hz, 1 H), 7.41 (d, *J*=1.43 Hz, 1 H), 7.40 (d, *J*=4.95 Hz, 1 H), 7.37 (dd, *J*=8.42, 2.26 Hz, 1 H), 7.32 (m, 4 H), 7.20 (m, 1 H), 4.35 (m, 2 H), 4.08 (m, 4 H), 3.73 (t, *J*=4.40 Hz, 2 H), 3.33 (s, 3 H)

### Step f : rel-(2aR)-3-chloro-2-[2-chloro-5-(3-phenylazetidin-3-yl)phenyl]-4-(2-methoxyethoxy)benzamide and rel-(2aS)-3-chloro-2-[2-chloro-5-(3-phenylazetidin-3-yl)phenyl]-4-(2-methoxyethoxy)benzamide

The compound was prepared using the same procedure detailed in example 1 step f starting from 3-chloro-2-[2-chloro-5-(3-phenylazetidin-3-yl)phenyl]-4-(2-methoxyethoxy)benzonitrile (379 mg, 0.836 mmol, 1.00 eq.) giving a mixture of two atropoisomers (221 mg, 0.469 mmol, 56.1 %). The two atropoisomers were separated by chiral chromatography (Column : Chiralpak IC, 250 *20mm*5µm, Mobile phase : 95% MtBE +0.1% NH3/ 5% MeOH +0.1% nPropylamine, Flowrate : 35ml/min, T° column : 10°C , λ = 260nm) to give **9a** rel-(2aR)-3-chloro-2-[2-chloro-5-(3-phenylazetidin-3-yl)phenyl]-4-(2-methoxyethoxy)benzamide (65 mg, 33.5 %) as a white powder. ¹H NMR (500 MHz, DMSO-*d₆*) δ ppm 7.49 (d, *J*=8.53 Hz, 1 H), 7.45 (br s, 1 H), 7.39 (d, *J*=8.39 Hz, 1 H), 7.31 (d, *J*=4.26 Hz, 4 H), 7.23 (d, *J*=8.67 Hz, 1 H), 7.18 (m, 3 H), 7.09 (br s, 1 H), 4.25 (m, 2 H), 4.09 (m, 2 H), 4.04 (d, *J*=7.43 Hz, 1 H), 4.00 (d, *J*=7.43 Hz, 1 H), 3.71 (t, *J*=4.54 Hz, 2 H), 3.33 (s, 3 H), [α]_{d}^{23.1} = +8.5° (C=0.43, DCM) ee (method D) 98.3% (Rt = 14.7 min); and **9b** rel-(2aS)-3-chloro-2-[2-chloro-5-(3-phenylazetidin-3-yl)phenyl]-4-(2-methoxyethoxy)benzamide (65 mg, 33.5 %) as a white powder. ¹H NMR (500 MHz, DMSO-*d₆*) δ ppm 7.49 (d, *J*=8.53 Hz, 1 H), 7.45 (br s, 1 H), 7.39 (d, *J*=8.39 Hz, 1 H), 7.31 (d, *J*=4.26 Hz, 4 H), 7.23 (d, *J*=8.67 Hz, 1 H), 7.18 (m, 3 H), 7.08 (br s, 1 H), 4.25 (m, 2 H), 4.09 (m, 2 H), 4.03 (d, *J*=7.43 Hz, 1 H), 3.99 (d, *J*=7.43 Hz, 1 H), 3.71 (t, *J*=4.54 Hz, 2 H), 3.33 (s, 3 H), [α]_{d}^{23.1} = -9.3° (C=0.44, DCM) ee (method D) 99.5% (Rt = 18.9 min).

### Example 10 : 2-[6-chloro-2-fluoro-3-[1-(2-hydroxyethyl)-3-phenyl-azetidin-3-yl]phenyl]-3-fluoro-4-(2-methoxyethoxy)benzamide

### Step a : 2-[6-chloro-2-fluoro-3-[1-(2-hydroxyethyl)-3-phenyl-azetidin-3-yl]phenyl]-3-fluoro-4-(2-methoxyethoxy)benzonitrile

To a 10 mL round bottom flask with a condenser, 2-[6-chloro-2-fluoro-3-(3-phenylazetidin-3-yl)phenyl]-3-fluoro-4-(2-methoxyethoxy)benzonitrile (Ex 4a, step d) (100 mg, 0.220 mmol, 1.0 eq.) was dissolved in acetonitrile (2.00 mL). K2CO3 (61.0 mg, 0.441 mmol, 2.01 eq.) and 2-bromoethanol (23.0 µL, 0.324 mmol, 1.47 eq.) were added at rt and the mixture was stirred under reflux for 1h30. The mixture was cooled to rt, was filtered and the solid was washed three times with ACN. The filtrate was evaporated to give 141 mg of a yellow paste. The crude product was purified by chromatography (Cartridge Biotage Sfär 5 g 20 µm silica, DCM-MeOH 100-0 / 2 CV, 100-0 to 95-5 / 10 CV, 95-5 / 10 CV, flow rate 18 ml / min) to give 2-[6-chloro-2-fluoro-3-[1-(2-hydroxyethyl)-3-phenyl-azetidin-3-yl]phenyl]-3-fluoro-4-(2-methoxyethoxy) benzonitrile (95.0 mg, 80.5 %) as a yellow gum. ¹H NMR (400 MHz, DMSO-*d₆*) δ ppm 7.86 (dd, *J*=8.75, 1.38 Hz, 1 H), 7.67 (m, 1 H), 7.57 (dd, J=8.47, 0.77 Hz, 1 H), 7.49 (t, *J*=8.53 Hz, 1 H), 7.45 (d, *J*=7.70 Hz, 2 H), 7.31 (m, 2 H), 7.21 (m, 1 H), 5.75 (s, 1 H), 4.34 (m, 2 H), 3.99 (d, *J*=7.26 Hz, 1 H), 3.93 (d, *J*=7.15 Hz, 1 H), 3.70 (m, 2 H), 3.67 (d, *J*=7.26 Hz, 1 H), 3.63 (d, *J*=7.15 Hz, 1 H), 3.38 (q, *J*=5.83 Hz, 2 H), 3.30 (s, 3 H), 2.51 (t, *J*=5.83 Hz, 2 H).

### Step b : 2-[6-chloro-2-fluoro-3-[1-(2-hydroxyethyl)-3-phenyl-azetidin-3-yl]phenyl]-3-fluoro-4-(2-methoxyethoxy)benzamide

The compound was prepared using the same procedure detailed in example 1 step f starting from 2-[6-chloro-2-fluoro-3-[1-(2-hydroxyethyl)-3-phenyl-azetidin-3-yl]phenyl]-3-fluoro-4-(2-methoxyethoxy)benzonitrile (91.0 mg, 0.182 mmol, 1.00 eq.) giving 2-[6-chloro-2-fluoro-3-[1-(2-hydroxyethyl)-3-phenyl-azetidin-3-yl]phenyl]-3-fluoro-4-(2-methoxyethoxy)benzamide (55.0 mg, 58.3 %) as a white powder. ¹H NMR (400 MHz, DMSO-*d₆*) δ ppm 7.70 (br s, 1 H), 7.51 (dd, *J*=8.64, 1.38 Hz, 1 H), 7.46 (d, *J*=7.48 Hz, 2 H), 7.38 (t, *J*=8.36 Hz, 1 H), 7.34 (d, *J*=8.25 Hz, 1 H), 7.30 (m, 3 H), 7.19 (tt, *J*=7.15, 1.32 Hz, 1 H), 7.09 (br s, 1 H), 4.25 (m, 2 H), 3.96 (d, *J*=7.15 Hz, 1 H), 3.88 (d, *J*=7.04 Hz, 1 H), 3.69 (m, 2 H), 3.62 (d, *J*=7.15 Hz, 1 H), 3.58 (d, *J*=7.04 Hz, 1 H), 3.38 (m, 2 H), 3.30 (s, 3 H), 2.51 (t, *J*=5.94 Hz, 2 H).

### Example 11a et 11b : rel-(2aR)-2-[6-chloro-2-fluoro-3-(3-phenylazetidin-3-yl)phenyl]-3-fluoro-4-(5-methylisoxazol-3-yl)oxy-benzamide and rel-(2aS)-2-[6-chloro-2-fluoro-3-(3-phenylazetidin-3-yl)phenyl]-3-fluoro-4-(5-methylisoxazol-3-yl)oxy-benzamide

### Step a: 2-bromo-3-fluoro-4-(5-methylisoxazol-3-yl)oxy-benzonitrile

At 0°C, in a 250ml round bottom flask, NaH (60% in oil) (23 mg, 0.5751 mmol, 1 eq.) was added to a solution of 5-methylisoxazol-3-ol (45 mg, 0.454 mmol, 1 eq.) in DMF (1.3 mL) under Ar. After 5 min, 2-bromo-3,4-difluorobenzonitrile (0.1 g, 0.5 mmol, 1 eq.) was added and the reaction mixture was stirred at 0°C for 30min. The reaction mixture was stirred at rt for 18h. The reaction mixture was quenched with a saturated solution of NH₄Cl and extracted with EtOAc (three times). The combined organic layers were washed with brine and dried over anhydrous Na₂SO₄. After filtration, the filtrate was concentrated under reduced pressure. The crude product was purified by chromatography (Column Biotage HC 10g (20µm), Flow : 30 ml/mn, Eluate A :cyclohexane eluate B: EtOAc, Gradient :A/B : 100/0 (iso) 3CV, then go to 90/10 in 4CV 90:10 (iso) 5cv then go to 80/20 in 3CV and 80/20 (iso) 15CV) to give 2-bromo-3-fluoro-4-(5-methylisoxazol-3-yl)oxy-benzonitrile (77 mg, 60%) as a white solid. ¹H NMR (500 MHz, DMSO-*d₆*) δ ppm 7.92 (dd, *J*=8.67, 1.65 Hz, 1 H), 7.71 (t, *J*=8.12 Hz, 1 H), 6.37 (s, 1 H), 2.40 (s, 3 H).

### Step b: 3-fluoro-4-(5-methylisoxazol-3-yl)oxy-2-trimethylstannyl-benzonitrile

The compound was prepared using the same procedure as detailed in example 19a step c starting from 2-bromo-3-fluoro-4-(5-methylisoxazol-3-yl)oxy-benzonitrile (244 mg, 0.821 mmol, 1 eq.) giving 3-fluoro-4-(5-methylisoxazol-3-yl)oxy-2-trimethylstannyl-benzonitrile (173 mg, 55.2%) as a colorless oil. ¹H NMR (400 MHz, DMSO-*d₆*) δ ppm 7.75 (dd, *J*=8.31, 0.61 Hz, 1 H), 7.56 (t, *J*=8.47 Hz, 1 H), 6.28 (q, *J*=0.81 Hz, 1 H), 2.39 (d, *J*=0.88 Hz, 3 H), 0.48 (s, 9 H).

### Step c: tert-butyl N-[2-[4-chloro-3-[6-cyano-2-fluoro-3-(5-methylisoxazol-3-yl)oxy-phenyl]-2-fluoro-phenyl]-2-hydroxy-2-phenyl-ethyl]-N-methyl-carbamate

### Step c : 3-(3-bromo-4-chloro-2-fluoro-phenyl)-3-phenyl-azetidine

The compound was prepared using the same procedure detailed in example 1 step e starting from tert-butyl 3-(3-bromo-4-chloro-2-fluoro-phenyl)-3-hydroxy-azetidine-1-carboxylate (Ex 4a, step b) (1.51 g, 3.97 mmol, 1.0 eq.) giving 3-(3-bromo-4-chloro-2-fluoro-phenyl)-3-phenyl-azetidine (492 mg, 46.6 %) as a beige powder, used without further prurification.

### Step d : tert-butyl 3-(3-bromo-4-chloro-2-fluoro-phenyl)-3-phenyl-azetidine-1-carboxylate

To a 10 mL round bottom flask,3-(3-bromo-4-chloro-2-fluoro-phenyl)-3-phenyl-azetidine (485 mg, 1.01 mmol, 1.0 eq.) and (BOC)2O (615 mg, 2.81 mmol, 2.79 eq.) were dissolved in dichloromethane (10 mL). DMAP (16 mg, 0.131 mmol, 0.13 eq.) and triethylamine (392 µL, 2.82 mmol, 2.79 eq.) were added and the mixture was stirred at rt for 2 h. The mixture was diluted with DCM and washed with 50 mL of aq. sat. NaHCO3. Aqueous layer was again extracted two times with DCM. The combined organic layers were dried over MgSO4, filtrated and concentrated under reduced pressure to give 755 mg of a yellow oil. The crude was purified by chromatography (Column Biotage HC 25g (60µm), cyclohexane / DCM, Flow rate 80 ml/mn) to give tert-butyl 3-(3-bromo-4-chloro-2-fluoro-phenyl)-3-phenyl-azetidine-1-carboxylate (333 mg, 74.7 %) as a white foam. ¹H NMR (400 MHz, DMSO-*d₆*) δ ppm 7.64 (m, 1 H), 7.53 (dd, *J*=8.53, 1.38 Hz, 1 H), 7.36 (d, *J*=4.51 Hz, 4 H), 7.26 (s, 1 H), 4.54 (m, 2 H), 4.39 (m, 2 H), 1.38 (s, 9 H).

### Step e : tert-butyl 3-[4-chloro-3-[6-cyano-2-fluoro-3-(5-methylisoxazol-3-yl)oxy-phenyl]-2-fluoro-phenyl]-3-phenyl-azetidine-1-carboxylate

The compound was prepared using the same procedure detailed in example 3a step e starting from tert-butyl 3-(3-bromo-4-chloro-2-fluoro-phenyl)-3-phenyl-azetidine-1-carboxylate (333 mg, 0.756 mmol, 1.0 eq.) and 3-fluoro-4-(5-methylisoxazol-3-yl)oxy-2-trimethylstannyl-benzonitrile (432 mg, 1.13 mmol, 1.5 eq.) giving tert-butyl 3-[4-chloro-3-[6-cyano-2-fluoro-3-(5-methylisoxazol-3-yl)oxy-phenyl]-2-fluoro-phenyl]-3-phenyl-azetidine-1 -carboxylate (215 mg, 49.2 %) as a colorless oil. ¹H NMR (400 MHz, DMSO-*d₆*) δ ppm 8.01 (dd, *J*=8.64, 1.38 Hz, 1 H), 7.84 (t, *J*=8.20 Hz, 2 H), 7.66 (dd, *J*=8.53, 0.94 Hz, 1 H), 7.36 (m, 4 H), 7.26 (m, 1 H), 6.37 (d, *J*=0.99 Hz, 1 H), 4.60 (d, *J*=9.24 Hz, 1 H), 4.52 (d, *J*=9.24 Hz, 1 H), 4.45 (d, *J*=9.02 Hz, 1 H), 4.34 (d, *J*=9.13 Hz, 1 H), 2.40 (d, *J*=0.88 Hz, 3 H), 1.39 (s, 9 H).

### Step f : tert-butyl 3-[3-[6-carbamoyl-2-fluoro-3-(5-methylisoxazol-3-yl)oxy-phenyl]-4-chloro-2-fluoro-phenyl]-3-phenyl-azetidine-1-carboxylate

The compound was prepared using the same procedure detailed in example 1 step f starting from tert-butyl 3-[4-chloro-3-[6-cyano-2-fluoro-3-(5-methylisoxazol-3-yl)oxy-phenyl]-2-fluorophenyl]-3-phenyl-azetidine-1-carboxylate (215 mg, 0.372 mmol, 1.00 eq.) giving tert-butyl 3-[3-[6-carbamoyl-2-fluoro-3-(5-methylisoxazol-3-yl)oxy-phenyl]-4-chloro-2-fluoro-phenyl]-3-phenyl-azetidine-1-carboxylate (142 mg, 64.1 %) as a white solid. ¹H NMR (400 MHz, DMSO-*d₆*) δ ppm 7.92 (br s, 1 H), 7.64 (t, *J*=8.47 Hz, 1 H), 7.58 (m, 2 H), 7.44 (dd, *J*=8.47, 0.88 Hz, 1 H), 7.34 (m, 5 H), 7.25 (m, 1 H), 6.24 (d, *J*=0.88 Hz, 1 H), 4.54 (d, *J*=8.91 Hz, 1 H), 4.46 (d, *J*=9.57 Hz, 1 H), 4.41 (d, *J*=9.35 Hz, 1 H), 4.25 (d, *J*=8.91 Hz, 1 H), 2.38 (d, *J*=0.88 Hz, 3 H), 1.40 (s, 9 H)

### Step g : rel-(2aR)-2-[6-chloro-2-fluoro-3-(3-phenylazetidin-3-yl)phenyl]-3-fluoro-4-(5-methylisoxazol-3-yl)oxy-benzamide and rel-(2aS)-2-[6-chloro-2-fluoro-3-(3-phenylazetidin-3-yl)phenyl]-3-fluoro-4-(5-methylisoxazol-3-yl)oxy-benzamide

To a 5 mL round bottom flask, tert-butyl 3-[3-[6-carbamoyl-2-fluoro-3-(5-methylisoxazol-3-yl)oxy-phenyl]-4-chloro-2-fluoro-phenyl]-3-phenyl-azetidine-1-carboxylate (140 mg, 0.23 mmol, 1.0 eq.) was dissolved in 1,4-dioxane (5.5 mL) at rt. Hydrochloric acid (4.0M solution in 1,4-dioxan (5.5 mL, 22 mmol, 94 eq.) was added. The resulting colorless mixture was stirred at rt for 7 h. The reaction mixture was concentrated to dryness to give a white foam, further triturated with Et2O and evaporated to give a mixture of two atropoisomers as a white solid. The two atropoisomers were separated by chirale chromatography (Column : Chiralpak IC, 250 *20mm*5µm, Mobile phase : 95% MtBE +0.1% NH3/ 5% MeOH +0.1% nPropylamine, Flowrate : 35ml/min, T° column : 10°C , λ = 260nm) to give 11a rel-(2aR)-2-[6-chloro-2-fluoro-3-(3-phenylazetidin-3-yl)phenyl]-3-fluoro-4-(5-methylisoxazol-3-yl)oxy-benzamide (28.6 mg, 24.6 %) as a white powder, ¹H NMR (500 MHz, DMSO-*d₆*) δ ppm 7.90 (s, 1 H), 7.63 (t, *J*=8.67 Hz, 1 H), 7.56 (dd, *J*=8.67, 0.83 Hz, 1 H), 7.45 (d, *J*=7.57 Hz, 2 H), 7.41 (m, 2 H), 7.31 (m, 3 H), 7.20 (tt, *J*=7.29, 1.00 Hz, 1 H), 6.24 (d, *J*=0.83 Hz, 1 H), 4.24 (d, *J*=7.98 Hz, 1 H), 4.21 (d, *J*=7.98 Hz, 1 H), 3.92 (d, *J*=7.43 Hz, 1 H), 3.80 (d, *J*=7.43 Hz, 1 H), 2.38 (d, *J*=0.83 Hz, 3 H), [α]_{d}^{21.2} = +16.0° (C=0.42, DCM) ee (method D) 99.5% (Rt = 13.1 min) and 11b rel-(2aS)-2-[6-chloro-2-fluoro-3-(3-phenylazetidin-3-yl)phenyl]-3-fluoro-4-(5-methylisoxazol-3-yl)oxy-benzamide (27 mg, 23.2 %), ¹H NMR (500 MHz, DMSO-*d₆*) δ ppm 7.89 (s, 1 H), 7.63 (t, *J*=8.67 Hz, 1 H), 7.56 (dd, *J*=8.67, 0.83 Hz, 1 H), 7.45 (d, *J*=7.43 Hz, 2 H), 7.41 (m, 2 H), 7.31 (m, 3 H), 7.20 (tt, *J*=7.29, 1.00 Hz, 1 H), 6.24 (d, *J*=0.96 Hz, 1 H), 4.23 (d, *J*=7.70 Hz, 1 H), 4.21 (d, *J*=7.70 Hz, 1 H), 3.92 (d, *J*=7.43 Hz, 1 H), 3.80 (d, *J*=7.43 Hz, 1 H), 2.38 (d, *J*=0.83 Hz, 3 H), [α]_{d}^{21.2} = -22.3° (C=0.39, DCM) ee (method D) 99.5% (Rt = 17.5 min).

### In-vitro Biochemical assay

The assay is based on a TR-FRET sandwich immunoassay using 6His-humanYAP⁶⁰⁻¹⁰⁰ and ⁻ FLAG-AVI-HumanTEAD4²¹⁷⁴³⁴. TEAD4 protein was produced using a process based on Hau et al., ChemBioChem, 2013, 14(10): 1218-1225. YAP was obtained via peptide synthesis. The compounds were dissolved at 50mM in 100% DMSO and serial dilutions were made in 100% DMSO. The diluted compound solutions were incubated at the same time than 6His-humanYAP⁶⁰⁻¹⁰⁰ (1.5nM in reaction buffer) and ⁻ FLAG-AVI-HumanTEAD4²¹⁷⁴³⁴ (1.5nM in reaction buffer) in white 384-well plates (Greiner) The final DMSO concentrations in the assay was 1%. The mix reaction was incubated for 120 min at room temperature. After 120 min of incubation, anti-His Europium cryptate labelled antibody and Streptavidin d2 were added to the reaction mix for 120 min more at room temperature. The plate was read with an Envision microplate reader. The data analyses were carried out by using the TR-FRET module. Two sequential measurements were carried out: at 620nm for the cryptate emission and at 665nm for the signal emitted by the d2 acceptor. The ratio of the two fluorescence intensities 665/620 enables the calculation of Delta F (%) which represents the relative energy transfer rate for each sample. The IC50 values were estimated by fitting the data by nonlinear fit regression (GraphPad Prism). The results are presented in Table 1.

**Table 1**

| **Example** | **FRET IC50 (µM)** |
|---|---|
| **1** | **14.4** |
| **2** | **188** |
| **3a** | **>200** |
| **3b** | **2.6** |
| **4a** | **50** |
| **4b** | **2.1** |
| **5** | **9** |
| **6a** | **57** |
| **6b** | **0.7** |
| **7a** | **87** |
| **7b** | **1.3** |

### Inhibition of malignant mesothelioma tumor cell growth

The tumor cell growth inhibitory activity of the compounds of formula (I) was evaluated in NCI-H2052 mesothelioma cell line harboring a NF2 mutation. 4,000 cells/well were plated in a 96-well black plate with clear flat bottom TC-Treated Imaging plate (Greiner Bio one #655090) in regular medium (as suggested from ATCC for each cell line) with serum, which was replaced the day after with starvation medium containing 1 % serum. After one day growth in the starvation medium, cells were incubated with the compounds. The starting concentration was 30 µM and serial dilutions in DMSO and medium were performed until 0.1µM to achieve a final DMSO concentration of 0.5%. The cells were then allowed to grow for 3 days, and then, EdU (Invitrogen, Molecular Probe) was added in each well at a final concentration of 10 µM and the cells were returned to the incubator for an additional 24h. The starvation medium was removed and 100 µl of PFA 4% containing Hoechst dye was adding in each well to fix the cells. Plates were then incubated at rt for 15 min, washed twice with PBS and the cells were permeabilized by adding 100 µL per well of triton-100 0.3%.

After 20 min cells were washed with PBS and EdU detection was performed according to the instructions of the manufacturer. Image acquisition was performed using the ImageXpress Micro and analyzed using the MetaXpress software (Molecular Device). Results were expressed as a percent of inhibition (%) of the cell proliferation values obtained with 0.5 % DMSO treatment alone. The cellular response was determined by fitting the concentration response curves using three parameters curve fit equation and determining the concentration that inhibited cell growth between 50% and 100%. The results are presented in Table 2.

**Table 2**

| **Example** | **H2052 IC50 (µM)** |
|---|---|
| **3a** | **32** |
| **3b** | **0.66** |
| **4b** | **0.40** |
| **5** | **5.4** |
| **6b** | **1.5** |

Aspects of the present disclosure are further illustrated by reference to the following, non-limiting embodiments.
1. A compound of formula (I): 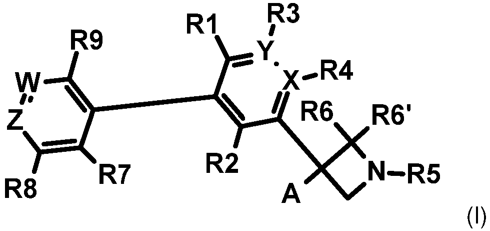 or a pharmaceutically acceptable salt, enantiomer, diastereomer, tautomer, solvate, isotope substituent, polymorph, prodrug or metabolite thereof, wherein:
   A is a phenyl optionally substituted with 1 to 3 substituents selected from halogen, (C₁-C₆)alkyl and (C₁-C₆)alkoxy; or a 4- to 7-membered unsaturated monocyclic group comprising from 1 to 3 heteroatoms, preferably 1 or 2 heteroatoms, chosen from nitrogen, oxygen and sulfur;;
   W and Z are each independently CR₁₅ or N;
   X and Y are each independently C or N;
   R₁ is H, halogen, (C₁-C₆)alkyl or (C₁-C₆)alkoxy;
   R₂ is H, halogen, (C₁-C₆)alkyl or (C₁-C₆)alkoxy;
   R₃ is absent when Y is N, or R₃ is H, halogen, -OH, -COCH₃, (C₁-C₆)alkyl or (C₁-C₆)alkoxy when Y is C;
   R₄ is absent when X is N, or R₄ is H, halogen, -OH, -COCH₃, (C₁-C₆)alkyl or (C₁-C₆)alkoxy when X is C;
   R₅ isH, (C₁-C₆)alkyl optionally substituted with halogen, -OH, -CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, - CONHR₁₂, -CONH₂, -NR₁₂R₁₃;
   R₆ and R₆' are each independently H, (C₁-C₆)alkyl or (C₃-C₆)cycloalkyl;
   Or R₆ and R₆' are bond together to form a 3 or 4 membered carbocyclic ring;
   R₇ is H, halogen, (C₁-C₆)alkyl or (C₁-C₆)alkoxy optionally substituted with (C₁-C₆)alkoxy;
   R₈ is H, halogen, (C₁-C₆)alkyl, (C₁-C₆)alkoxy, (C₁-C₆)alkoxy optionally substituted with (C₁-C₆)alkoxy,
   -OR₁₀, -OCH₂R₁₀, -NHR₁₀, -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₉ is (C₁-C₆)alkyl, (C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₁ is H, -OH ,-NH₂,(C₁-C₆)alkyl, (C₁-C₆)alkoxy, and -NHR₁₄ ;
   R₁₂ is H or (C₁-C₆)alkyl;
   R₁₃ is H or (C₁-C₆)alkyl;
   R₁₄ is (C₁-C₆)alkyl;
   R₁₅ is H, halogen, (C₁-C₆)alkyl or (C₁-C₆)alkoxy
2. The compound of item 1, or a pharmaceutically acceptable salt thereof, wherein A is a phenyl optionally substituted with 1 to 3 substituents selected from halogen, (C₁-C₆)alkyl and (C₁-C₆)alkoxy.
3. The compound of item 1, or a pharmaceutically acceptable salt thereof, wherein A is a 4- to 7-membered unsaturated, monocyclic group comprising from 1 to 3 heteroatoms, preferably 1 or 2 heteroatoms, chosen from nitrogen, oxygen and sulfur.
4. The compound of any preceding item, or a pharmaceutically acceptable salt, enantiomer, diastereomer, tautomer, solvate, isotope substituent, polymorph, prodrug or metabolite thereof, wherein W and Z are each CH.
5. The compound of any preceding item, or a pharmaceutically acceptable salt, enantiomer, diastereomer, tautomer, solvate, isotope substituent, polymorph, prodrug or metabolite thereof, wherein X and Y are each C.
6. The compound of any preceding item, or a pharmaceutically acceptable salt, enantiomer, diastereomer, tautomer, solvate, isotope substituent, polymorph, prodrug or metabolite thereof, wherein R₁ is halogen or (C₁-C₆)alkyl.
7. The compound of any preceding item, or a pharmaceutically acceptable salt, enantiomer, diastereomer, tautomer, solvate, isotope substituent, polymorph, prodrug or metabolite thereof, wherein R₂ is H, halogen or (C₁-C₆)alkyl.
8. The compound of any preceding item, or a pharmaceutically acceptable salt, enantiomer, diastereomer, tautomer, solvate, isotope substituent, polymorph, prodrug or metabolite thereof, wherein R₃ when present is H or halogen.
9. The compound of any preceding item, or a pharmaceutically acceptable salt, enantiomer, diastereomer, tautomer, solvate, isotope substituent, polymorph, prodrug or metabolite thereof, wherein R₄ when present is H.
10. The compound of any preceding item, or a pharmaceutically acceptable salt, enantiomer, diastereomer, tautomer, solvate, isotope substituent, polymorph, prodrug or metabolite thereof, wherein R₆ and R'₆ are each H.
11. The compound of any preceding item, or a pharmaceutically acceptable salt, enantiomer, diastereomer, tautomer, solvate, isotope substituent, polymorph, prodrug or metabolite thereof, wherein R₅ is H or (C₁-C₆)alkyl.
12. The compound of any preceding item, or a pharmaceutically acceptable salt, enantiomer, diastereomer, tautomer, solvate, isotope substituent, polymorph, prodrug or metabolite thereof, wherein R₇ is halogen or (C₁-C₆)alkyl.
13. The compound of any preceding item, or a pharmaceutically acceptable salt, enantiomer, diastereomer, tautomer, solvate, isotope substituent, polymorph, prodrug or metabolite thereof, wherein R₈ is H or (C₁-C₆)alkoxy optionally substituted with (C₁-C₆)alkoxy.
14. The compound of any preceding item, or a pharmaceutically acceptable salt, enantiomer, diastereomer, tautomer, solvate, isotope substituent, polymorph, prodrug or metabolite thereof, wherein R₁₁ is H or (C₁-C₆)alkyl.
15. The compound of item 1, which is selected from:
   2-[2-chloro-5-(3-phenylazetidin-3-yl)phenyl]-3-fluoro-4-(2-methoxyethoxy)benzamide;
   2-[2-chloro-5-(3-phenylazetidin-3-yl)phenyl]-3-methyl-benzamide;
   rel-(2aR)-2-[6-chloro-2-methyl-3-(3-phenylazetidin-3-yl)phenyl]-3-fluoro-4-(2-methoxyethoxy)benzamide;
   rel-(2aS)-2-[6-chloro-2-methyl-3-(3-phenylazetidin-3-yl)phenyl]-3-fluoro-4-(2-methoxyethoxy)benzamide;
   rel-(2aR)-2-[6-chloro-2-fluoro-3-(3-phenylazetidin-3-yl)phenyl]-3-fluoro-4-(2-methoxyethoxy)benzamide;
   rel-(2aS)-2-[6-chloro-2-fluoro-3-(3-phenylazetidin-3-yl)phenyl]-3-fluoro-4-(2-methoxyethoxy)benzamide;
   2-[2-chloro-5-(1-methyl-3-phenyl-azetidin-3-yl)phenyl]-3-fluoro-4-(2-methoxyethoxy)benzamide;
   rel-(2aR)-2-[2,3-dichloro-6-fluoro-5-(3-phenylazetidin-3-yl)phenyl]-3-fluoro-4-(2-methoxyethoxy)benzamide;
   rel-(2aS)-2-[2,3-dichloro-6-fluoro-5-(3-phenylazetidin-3-yl)phenyl]-3-fluoro-4-(2-methoxyethoxy)benzamide;
   and pharmaceutically acceptable salts, enantiomers, diastereomers, tautomers, solvates, isotope substituents, polymorphs, prodrugs or metabolites thereof.
16. A pharmaceutical composition comprising a compound according to any one of items 1 to 15, or a pharmaceutically acceptable salt, enantiomer, diastereomer, tautomer, solvate, isotope substituent, polymorph, prodrug or metabolite thereof, and at least one pharmaceutically acceptable excipient.
17. The pharmaceutical composition of item 16, which further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and combinations thereof.
18. The pharmaceutical composition according to item 16 or item 17, for use for the prevention or the treatment of a cancer.
19. The pharmaceutical composition according to item 16 or item 17, for use for the prevention or the treatment of a fibrosis related disease.
20. A compound according to any one of items 1 to 15, or a pharmaceutically acceptable salt, enantiomer, diastereomer, tautomer, solvate, isotope substituent, polymorph, prodrug or metabolite thereof, for use as a medicament.
21. A compound according to any one of items 1 to 15, or a pharmaceutically acceptable salt, enantiomer, diastereomer, tautomer, solvate, isotope substituent, polymorph, prodrug or metabolite thereof, for use for the treatment of a cancer.
22. A compound according to any one of items 1 to 15, or a pharmaceutically acceptable salt, enantiomer, diastereomer, tautomer, solvate, isotope substituent, polymorph, prodrug or metabolite thereof, for use for the prevention of a cancer.
23. Use of a compound according to any one of items 1 to 15, or a pharmaceutically acceptable salt, enantiomer, diastereomer, tautomer, solvate, isotope substituent, polymorph, prodrug or metabolite thereof, in the manufacture of a medicament for the treatment of a cancer.
24. Use of a compound according to any one of items 1 to 15, or a pharmaceutically acceptable salt, enantiomer, diastereomer, tautomer, solvate, isotope substituent, polymorph, prodrug or metabolite thereof, in the manufacture of a medicament for the prevention of a cancer.
25. A method of treating a cancer in a patient, comprising administering to the patient an effective amount of a compound of formula (I) according to any one of items 1 to 15, or a pharmaceutically acceptable salt, enantiomer, diastereomer, tautomer, solvate, isotope substituent, polymorph, prodrug or metabolite thereof.
26. A method of preventing a cancer in a patient, comprising administering to the patient an effective amount of a compound of formula (I) according to any one of items 1 to 15, or a pharmaceutically acceptable salt, enantiomer, diastereomer, tautomer, solvate, isotope substituent, polymorph, prodrug or metabolite thereof.
27. The method according to item 25 or item 26, which further comprises administering to the patient at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and combinations thereof.
28. The compound for use, use or method according to any one of items 20 to 27, wherein the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.
29. A compound according to any one of items 1 to 15, or a pharmaceutically acceptable salt, enantiomer, diastereomer, tautomer, solvate, isotope substituent, polymorph, prodrug or metabolite thereof, for use for the treatment of a fibrosis related disease.
30. A compound according to any one of items 1 to 15, or a pharmaceutically acceptable salt, enantiomer, diastereomer, tautomer, solvate, isotope substituent, polymorph, prodrug or metabolite thereof, for use for the prevention of a fibrosis related disease.
31. Use of a compound according to any one of items 1 to 15, or a pharmaceutically acceptable salt, enantiomer, diastereomer, tautomer, solvate, isotope substituent, polymorph, prodrug or metabolite thereof, in the manufacture of a medicament for the treatment of a fibrosis related disease.
32. Use of a compound according to any one of items 1 to 15, or a pharmaceutically acceptable salt, enantiomer, diastereomer, tautomer, solvate, isotope substituent, polymorph, prodrug or metabolite thereof, in the manufacture of a medicament for the prevention of a fibrosis related disease.
33. A method of treating a fibrosis related disease in a patient, comprising administering to the patient an effective amount of a compound of formula (I) according to any one of items 1 to 15, or a pharmaceutically acceptable salt, enantiomer, diastereomer, tautomer, solvate, isotope substituent, polymorph, prodrug or metabolite thereof.
34. A method of preventing a fibrosis related disease in a patient, comprising administering to the patient an effective amount of a compound of formula (I) according to any one of items 1 to 15, or a pharmaceutically acceptable salt, enantiomer, diastereomer, tautomer, solvate, isotope substituent, polymorph, prodrug or metabolite thereof.
35. The compound for use, use or method according to any one of items 29 to 34, wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease).
36. A pharmaceutical composition according to item 16 or item 17, for use as a medicament.
37. Use of a pharmaceutical composition according to item 16 or item 17 in the manufacture of a medicament for the treatment or prevention of a cancer.
38. A method of treating or preventing a cancer in a patient, comprising administering to the patient a pharmaceutical composition according to item 16, wherein the pharmaceutical composition comprises an effective amount of a compound of formula (I) or a pharmaceutically acceptable salt, enantiomer, diastereomer, tautomer, solvate, isotope substituent, polymorph, prodrug or metabolite thereof.
39. The method according to item 38, which further comprises administering to the patient at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic and combinations thereof.
40. The pharmaceutical composition for use, use or method according to any one of items 18 and 37 to 39, wherein the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.
41. Use of a pharmaceutical composition according to item 16 in the manufacture of a medicament for the treatment or prevention of a fibrosis related disease.
42. A method of treating or preventing a fibrosis related disease in a patient, comprising administering to the patient a pharmaceutical composition according to item 16, wherein the pharmaceutical composition comprises an effective amount of a compound of formula (I) or a pharmaceutically acceptable salt, enantiomer, diastereomer, tautomer, solvate, isotope substituent, polymorph, prodrug or metabolite thereof.
43. The pharmaceutical composition for use, use or method according to any one of items 19, 41 or 42, wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease).
44. A method of modulating one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
45. A method of inhibiting one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
46. A method of activating one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
47. A compound of formula (I) according to any one of items 1 to 15 or a pharmaceutically acceptable salt thereof, or the pharmaceutical composition, enantiomer, diastereomer, tautomer, solvates, isotope substituent, polymorph, prodrug or metabolite thereof as described herein, for treating or preventing a disease or disorder by modulating or inhibiting or activating one or more of proteins encompassed by, or related to, the Hippo pathway, in a subject in need thereof.
48. Uses of a compound of formula (I) according to any one of items 1 to 15 or a pharmaceutically acceptable salt thereof, or the pharmaceutical composition, enantiomer, diastereomer, tautomer, solvate, isotope substituent, polymorph, prodrug or metabolite as described herein, in the preparation of a medicament for treating or preventing a disease or disorder by modulating or inhibiting or activating one or more of proteins encompassed by, or related to, the Hippo pathway, in a subject in need thereof.

## Claims

1. A compound of formula (I): 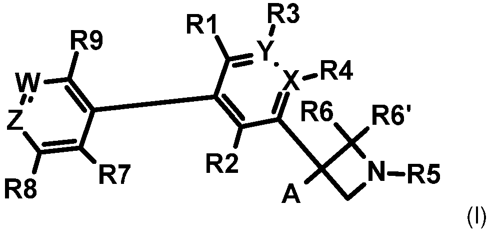 or a pharmaceutically acceptable salt, enantiomer, diastereomer, tautomer, solvate, isotope substituent, polymorph, prodrug or metabolite thereof, wherein:
A is a phenyl optionally substituted with 1 to 3 substituents selected from halogen, (C₁-C₆)alkyl and (C₁-C₆)alkoxy; or a 4- to 7-membered unsaturated monocyclic group comprising from 1 to 3 heteroatoms, preferably 1 or 2 heteroatoms, chosen from nitrogen, oxygen and sulfur;;
W and Z are each independently CR₁₅ or N;
X and Y are each independently C or N;
R₁ is H, halogen, (C₁-C₆)alkyl or (C₁-C₆)alkoxy;
R₂ is H, halogen, (C₁-C₆)alkyl or (C₁-C₆)alkoxy;
R₃ is absent when Y is N, or R₃ is H, halogen, -OH, -COCH₃, (C₁-C₆)alkyl or (C₁-C₆)alkoxy when Y is C;
R₄ is absent when X is N, or R₄ is H, halogen, -OH, -COCH₃, (C₁-C₆)alkyl or (C₁-C₆)alkoxy when X is C;
R₅ is H, (C₁-C₆)alkyl optionally substituted with halogen, -OH, -CONHR₁₂, -CONH₂, - NR₁₂R₁₃, (C₁-C₆)alkoxy or (C₃-C₆)cycloalkyl; or (C₃-C₆)cycloalkyl optionally substituted with - OR₁₂, -CONHR₁₂, -CONH₂, -NR₁₂R₁₃;
R₆ and R₆' are each independently H, (C₁-C₆)alkyl or (C₃-C₆)cycloalkyl;
Or R₆ and R₆' are bond together to form a 3 or 4 membered carbocyclic ring;
R₇ is H, halogen, (C₁-C₆)alkyl or (C₁-C₆)alkoxy;
R₈ is H, halogen, (C₁-C₆)alkyl, (C₁-C₆)alkoxy, (C₁-C₆)alkoxy optionally substituted with (C₁-C₆)alkoxy, -OR₁₀, -OCH₂R₁₀, -NHR₁₀, -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₉ is (C₁-C₆)alkyl , (C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₁ is H, -OH ,-NH₂,(C₁-C₆)alkyl, (C₁-C₆)alkoxy, and -NHR₁₄ ;
R₁₂ is H or (C₁-C₆)alkyl;
R₁₃ is H or (C₁-C₆)alkyl;
R₁₄ is (C₁-C₆)alkyl;
R₁₅ is H, halogen, (C₁-C₆)alkyl or (C₁-C₆)alkoxy. .

2. The compound of claim 1, or a pharmaceutically acceptable salt, enantiomer, diastereomer, tautomer, solvate, isotope substituent, polymorph, prodrug or metabolite thereof, wherein A is a phenyl optionally substituted with 1 to 3 substituents selected from halogen, (C₁-C₆)alkyl and (C₁-C₆)alkoxy.

3. The compound of claim 1 or claim 2, or a pharmaceutically acceptable salt, enantiomer, diastereomer, tautomer, solvate, isotope substituent, polymorph, prodrug or metabolite thereof, wherein W and Z are each CH.

4. The compound of any preceding claim, or a pharmaceutically acceptable salt, enantiomer, diastereomer, tautomer, solvate, isotope substituent, polymorph, prodrug or metabolite thereof, wherein X and Y are each C.

5. The compound of any preceding claim, or a pharmaceutically acceptable salt, enantiomer, diastereomer, tautomer, solvate, isotope substituent, polymorph, prodrug or metabolite thereof, wherein R₁ is halogen.

6. The compound of any preceding claim, or a pharmaceutically acceptable salt, enantiomer, diastereomer, tautomer, solvate, isotope substituent, polymorph, prodrug or metabolite thereof, wherein R₂ is H, halogen or (C₁-C₆)alkyl.

7. The compound of any preceding claim, or a pharmaceutically acceptable salt, enantiomer, diastereomer, tautomer, solvate, isotope substituent, polymorph, prodrug or metabolite thereof, wherein R₃ when present is H or halogen.

8. The compound of any preceding claim, or a pharmaceutically acceptable salt, enantiomer, diastereomer, tautomer, solvate, isotope substituent, polymorph, prodrug or metabolite thereof, wherein R₄ when present is H.

9. The compound of any preceding claim, or a pharmaceutically acceptable salt, enantiomer, diastereomer, tautomer, solvate, isotope substituent, polymorph, prodrug or metabolite thereof, wherein R₆ and R'₆ are each H.

10. The compound of any preceding claim, or a pharmaceutically acceptable salt, enantiomer, diastereomer, tautomer, solvate, isotope substituent, polymorph, prodrug or metabolite thereof, wherein R₅ is H or (C₁-C₆)alkyl.

11. The compound of any preceding claim, or a pharmaceutically acceptable salt, enantiomer, diastereomer, tautomer, solvate, isotope substituent, polymorph, prodrug or metabolite thereof, wherein R₇ is halogen or (C₁-C₆)alkyl.

12. The compound of any preceding claim, or a pharmaceutically acceptable salt, enantiomer, diastereomer, tautomer, solvate, isotope substituent, polymorph, prodrug or metabolite thereof, wherein R₈ is H or (C₁-C₆)alkoxy optionally substituted with (C₁-C₆)alkoxy.

13. The compound of claim 1, which is selected from:
2-[2-chloro-5-(3-phenylazetidin-3-yl)phenyl]-3-fluoro-4-(2-methoxyethoxy)benzamide;
2-[2-chloro-5-(3-phenylazetidin-3-yl)phenyl]-3-methyl-benzamide;
rel-(2aR)-2-[6-chloro-2-methyl-3-(3-phenylazetidin-3-yl)phenyl]-3-fluoro-4-(2-methoxyethoxy)benzamide;
rel-(2aS)-2-[6-chloro-2-methyl-3-(3-phenylazetidin-3-yl)phenyl]-3-fluoro-4-(2-methoxyethoxy)benzamide;
rel-(2aR)-2-[6-chloro-2-fluoro-3-(3-phenylazetidin-3-yl)phenyl]-3-fluoro-4-(2-methoxyethoxy)benzamide;
rel-(2aS)-2-[6-chloro-2-fluoro-3-(3-phenylazetidin-3-yl)phenyl]-3-fluoro-4-(2-methoxyethoxy)benzamide;
2-[2-chloro-5-(1-methyl-3-phenyl-azetidin-3-yl)phenyl]-3-fluoro-4-(2-methoxyethoxy)benzamide;
rel-(2aR)-2-[2,3-dichloro-6-fluoro-5-(3-phenylazetidin-3-yl)phenyl]-3-fluoro-4-(2-methoxyethoxy)benzamide;
rel-(2aS)-2-[2,3-dichloro-6-fluoro-5-(3-phenylazetidin-3-yl)phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

14. The compound of claim 1, which is selected from:
2-[2-chloro-5-(3-phenylazetidin-3-yl)phenyl]-3-fluoro-4-(2-methoxyethoxy)benzamide;
rel-(2aS)-2-[6-chloro-2-methyl-3-(3-phenylazetidin-3-yl)phenyl]-3-fluoro-4-(2-methoxyethoxy)benzamide;
rel-(2aR)-2-[6-chloro-2-fluoro-3-(3-phenylazetidin-3-yl)phenyl]-3-fluoro-4-(2-methoxyethoxy)benzamide;
2-[2-chloro-5-(1-methyl-3-phenyl-azetidin-3-yl)phenyl]-3-fluoro-4-(2-methoxyethoxy)benzamide;
rel-(2aS)-2-[2,3-dichloro-6-fluoro-5-(3-phenylazetidin-3-yl)phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

15. A pharmaceutical composition comprising a compound according to any preceding claim, or a pharmaceutically acceptable salt, enantiomer, diastereomer, tautomer, solvate, isotope substituent, polymorph, prodrug or metabolite, and one or more pharmaceutically acceptable excipient(s).

16. The pharmaceutical composition of claim 15, which further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

17. The compound of any one of claims 1 to 14, or a pharmaceutically acceptable salt, enantiomer, diastereomer, tautomer, solvate, isotope substituent, polymorph, prodrug or metabolite thereof, for use as a medicament.

18. The compound of any one of claims 1 to 14, or a pharmaceutically acceptable salt, enantiomer, diastereomer, tautomer, solvate, isotope substituent, polymorph, prodrug or metabolite thereof, for use for the treatment or prevention of a cancer or of a fibrosis related disease.

19. The compound for use of claim 18, wherein the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.

20. The compound for use of claim 18, wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease)..

21. A method of modulating one or more of proteins encompassed by, or related to, the Hippo pathway in a subject, comprising administering to a subject in need thereof a compound of formula (I) of any one of claims 1 to 14, or the pharmaceutical composition according to any one of claims 15 to 16, or a pharmaceutically acceptable salt, enantiomer, diastereomer, tautomer, solvate, isotope substituent, polymorph, prodrug or metabolite thereof.

22. A compound of formula (I) according to any one of claims 1 to 14 or a pharmaceutically acceptable salt thereof, or the pharmaceutical composition according to any one of claims 15 to 16 or, enantiomer, diastereomer, tautomer, solvate, isotope substituent, polymorph, prodrug or metabolite thereof, for treating or preventing a disease or disorder by modulating or inhibiting or activating one or more of proteins encompassed by, or related to, the Hippo pathway, in a subject in need thereof.

23. Uses of a compound of formula (I) according to any one of claims 1 to 13 or a pharmaceutically acceptable salt thereof, or the pharmaceutical composition according to any one of claims 15 to 16 or, enantiomer, diastereomer, tautomer, solvate, isotope substituent, polymorph, prodrug or metabolite thereof, in the preparation of a medicament for treating or preventing a disease or disorder by modulating or inhibiting or activating one or more of proteins encompassed by, or related to, the Hippo pathway, in a subject in need thereof.
